Europäisches Patentamt

European Patent Office

Office européen des brevets

(11) Publication number: **0 270 977 B1**

(12) **EUROPEAN PATENT SPECIFICATION**

(45) Date of publication of patent specification: **19.02.92**  (51) Int. Cl.5: **A61K 7/16**

(21) Application number: **87117706.9**

(22) Date of filing: **30.11.87**

(54) **Anticariogenic compositions.**

(30) Priority: **02.12.86 US 937196**

(43) Date of publication of application:
**15.06.88 Bulletin 88/24**

(45) Publication of the grant of the patent:
**19.02.92 Bulletin 92/08**

(84) Designated Contracting States:
**BE CH DE ES FR GB IT LI NL SE**

(56) References cited:
**US-A- 4 108 979**
**US-A- 4 146 605**
**US-A- 4 645 662**

(73) Proprietor: **PURDUE RESEARCH FOUNDATION**
**328 Enad Building, Purdue University**
**West Lafayette, IN 47907(US)**

(72) Inventor: **Kleber, Carl J.**
**8331 Tewksbury Court**
**Fort Wayne Indiana 46835(US)**
Inventor: **Putt, Mark S.**
**3311 Cabot Lane**
**Fort Wayne Indiana 46805(US)**

(74) Representative: **Kraus, Walter, Dr. et al**
**Patentanwälte Kraus, Weisert & Partner**
**Thomas-Wimmer-Ring 15**
**W-8000 München 22(DE)**

EP 0 270 977 B1

Rank Xerox (UK) Business Services

## Description

This invention relates to an oral preparation for use in the prevention of dental caries. More particularly, this invention relates to a stable, low-pH emulsion for oral administration as a dental caries prophylactic.

It has been found that aluminum salts can be used in a flavored emulsion with selected nonionic surfactants to produce a cariostatically active low-pH composition that is both stable and palatable to humans. The oral composition may be used in the preparation of a wide variety of products, including mouthwashes, chewing gums, prophylaxis pastes, dentifrices, dental rinses, and lozenges.

Anticariogenic preparations are well known in the art. Topical formulations containing fluoride, stannous fluoride, or sodium fluoride, for example, are known to provide partial protection against dental caries. Although effective dental caries protection has been obtained through the use of such fluoride-containing compounds, occasional side effects have been experienced with certain of the known fluoride-containing anticariogenic agents, particularly certain tin-containing salts. A brownish pigmentation has been noted after anticariogenic agents containing the stannous ion have been applied to the teeth. Although the pigmentation is not necessarily undesirable from a physiological standpoint, for aesthetic reasons, it would be desirable to provide an effective anticariogenic agent that does not discolor enamel.

The utility of fluoride materials has also been limited by the extent of their solubility and stability in aqueous media. For example, sodium fluoride is only soluble to the extent of about 4% in water. Furthermore, because of toxicity concerns, current regulations imposed by the U.S. Food and Drug Administration limit the amount of fluoride that can be provided in products sold for over-the-counter use.

For the foregoing and other reasons, dental researchers have continued their efforts to develop new anticariogenic agents which not only demonstrate a high level of anticariogenic effectiveness but are non-toxic, stable, and widely available. It has been suggested that aluminum salts may have a beneficial effect in reducing dental caries or in facilitating the uptake of fluoride by the dental enamel. See, e.g., Manly, et al., J. Dent. Res. 28: 160 (1948); Regolati, et al., Helv. Odont. Acta. 13: 59 (1969); and Kelada, "Electrochemical Characteristics of Free and Complexed Fluorides in drinking Water and the Effects of Aluminum and Iron on Fluoride Incorporation Into Tooth Enamel," Univ. Michigan Thesis (1972).

In vitro studies have shown that pretreatment of enamel with aluminum solutions resulted in increased fluoride uptake when followed by treatment with a fluoride solution; however, treatment with combinations of aluminum and fluoride did not afford any added benefit over that of fluoride alone. McCann, Arch. Oral Biol 14:521 (1969); and Gerhardt, et al., J. Dent Res 51:870 (1972). The foregoing techniques dealt primarily with the use of aluminum in combination with fluorides and did not focus on the effect of aluminum in the absence of fluoride.

Nor has the use of aluminum salts in dentifrices demonstrated a desirable result, primarily because there has been but recent recognition that conventional dentifrice abrasives are incompatible with sources of biologically available aluminum US-A-4 108 979. While FR-A-3610M describes a specific combination of aluminum lactate, aluminum fluoride, and calcium pyrophosphate, the abrasive interferes with aluminum ion activity by forming insoluble aluminum phosphate. Similarly, US-A-3 095 356 uses aluminum salts such as aluminum fluoride to coact with insoluble sodium metaphosphate abrasives to reduce the solubility of such abrasives and to increase fluoride uptake, but without independent therapeutic advantage being taken of the aluminum.

Canadian Pat. No. 829,272 describes acidic dentifrices comprising a combination of surface active substances and albumen-coagulating substances such as certain carboxylic acid salts of aluminum and other metals. However, this patent fails to teach that the satisfactory use of aluminum ions in dentifrices is dependent upon the use of aluminum compatible constituents, that is, constituents which when present in solution with aluminum ions, do not complex or react with them to render said ions unreactive with the surface of teeth.

US-A-4 108 981 describes an alkaline mouthwash composition (pH 7-9) comprising aluminum salts and carboxylic acid. However, that patent teaches that the carboxylic acid is required to stabilize the aluminum in the mouthwash preparation. Similarly US-A-4 153 732 teaches that ascorbic and adipic acids can be used to stabilize an aluminum-containing comestible. It further teaches that, in fact, many carboxylic acids interfere with aluminum ions. (Column 5; lines 7-9). There is nothing to suggest the use of aluminum ions without a stabilizing acid, merely that certain carboxylic acids are more compatible with aluminum.

In sum, the prior art has not heretofore suggested a stable anticariogenic preparation of aluminum ions at low pH without the presence of carboxylic acids and/or fluoride. One of the main problems associated with the formulation of such aluminum ion preparations is that aluminum is naturally very astringent, tart-tasting, and produces a profound drying sensation in the oral cavity. Another problem is that aluminum is very reactive and can easily be inactivated by many conventional cosmetic ingredients.

An object of the present invention therefore is to provide a novel, low-pH composition for inhibiting dental caries employing aluminum in a compatible emulsion system.

A further object is to provide cariostatically effective aluminum-containing oral preparations which are both stable and palatable.

A still further object is to provide new anticariogenic emulsions useful for preparation of mouthwashes, dentifrices, chewing gums, lozenges, and prophylaxis pastes.

The present invention is directed to anticariogenic aluminum-containing emulsions having a pH of 2.5 to 5.0 and adapted for application to teeth. The compositions comprise water-soluble aluminum salts in an amount sufficient to provide an anticariogenic concentration of aluminum ions in an aqueous emulsion stabilized with selected surfactants. Preferably for enhanced palatability the present compositions include, in addition, one or more substances selected from flavor oils, humectants and sweeteners.

Cariostatically active emulsions of this invention may be prepared by a variety of methods so long as aluminum ions are provided in an aqueous medium having a pH in the range of 2.5 to 5.0. At pH levels less than 2.5, the emulsion is generally too astringent-tasting, causes erosion of the teeth, and is generally not palatable. Above pH 5.0, substantially all of the aluminum is precipitated as aluminum hydroxide. A preferred pH range for the present formulation is 3.0 to 4.5, and more preferably 3.5 to 3.9.

The invention features water-soluble aluminum salts in an emulsion. The particular water-soluble aluminum salt employed is not critical, and substantially any nontoxic, water-soluble aluminum ion-containing salt may be used. Suitable water-soluble aluminum salts include aluminum potassium sulfate, aluminum chloride, aluminum sodium sulfate, aluminum sodium phosphate, aluminum sulfate, aluminum nitrate, sodium aluminate, and mixtures thereof. Other water-soluble aluminum salts are aluminum acetate, aluminum ammonium sulfate, aluminum bromate, aluminum bromide, aluminum chlorate, aluminum iodide, aluminum lactate, aluminum phenylsulfonate, and potassium aluminate. Aluminum potassium sulfate and aluminum chloride are preferred by reason of their wide availability and well-established safety. The aluminum salts are present in an amount sufficient to provide an emulsion having a concentration of available aluminum ions of 10 to 50 000 ppm, preferably 250 to 10 000 ppm and particularly for mouthwash formulations, most preferably 500 to 2000 ppm. "Available aluminum ions" for the purpose of defining this invention are aluminum ions capable of reacting or complexing with a substance of the teeth, including enamel, cementum and dentin. Aluminum ions which are complexed with anionic/chelating substances exhibit little if any reactivity with tooth surfaces.

Representative humectant materials useful in this invention include glycerin, mannitol, sorbitol, xylitol, sugar alcohols, and mixtures thereof. Glycerin is the preferred humectant on the basis of cost, availability, and ability to reduce the astringency of the aluminum. Humectant materials are present in the emulsion in an amount ranging from 1 to 90 percent by weight, preferably 5 to 60 percent by weight, and more preferably 5 to 20 percent by weight. In mouthwash formulations, a humectant is typically used at 10 percent by weight of the formulation.

The emulsion also includes a sweetening agent. Suitable sweeteners include sucrose, fructose, levulose, and dextrose, and mixtures thereof as well as noncariogenic artificial sweeteners such as saccharin, cyclamate and aspartame. Preferably a noncariogenic sweetener is employed in the oral composition of this invention.

Due to the astringent-tasting uncomplexed aluminum compounds, it is typically necessary to utilize a flavoring material to mask effectively the astringent taste. Representative flavoring oils include oils of wintergreen, peppermint, citrus, cassia, cherry, tutti frutti, raspberry, root beer, orange, grape, and other suitable flavor oils. The flavor oils are present in concentrations ranging from 0.1 to 0.5 percent by weight and preferably, 0.2 to 2.0 percent by weight. For most formulations, no more than 1.0 weight percent flavor oil is required to obtain acceptable palatability.

The emulsions of this invention are predicated on the discovery that certain surfactants, especially nonionic hydrophilic surfactants, enable formulation of stable, low-pH, flavor oil-aqueous aluminum salt emulsions. The use of such aluminum-compatible surfactants enables the formulation of stable, aqueous systems containing high flavor oil levels, thereby eliminating the need of ethyl alcohol for flavor oil solubilization. Nonionic surfactants, in general, tend to decompose at alkaline pH. They are, however, quite stable under acidic conditions, as is the case for the aluminum compositions of this invention.

Certain classes of cationic and anionic surfactants have also been demonstrated to exhibit surprising compatibility and efficacy in the anticariogenic compositions of this invention. Thus aluminum ions have also been found to retain "activity" in the presence of art-recognized polyalkoxylated, e.g., polyethoxylated and polypropoxylated surfactants, particularly polyalkoxy carboxylates, polyalkoxy sulfates and per-alkoxylated amines. Polyalkoxylated anionic and cationic surfactants possess a sufficient and apparently dominant nonionic character which manifests itself in an unexpected level of compatibility with cationic aluminum. Quarternary ammonium surfactants and amphoteric surfactants derived from the amino acids glycine, cysteine and phenylalanine have also exhibited acceptable compatibility with aluminum ions.

The surfactants used for an aluminum rinse in accordance with this invention are non-toxic and water-soluble, each preferably with a dominant hydrophilic moiety. The hydrophilic surfactants are nonionic or have the aluminum-compatible functionality of nonionic surfactants.

One suitable hydrophilic nonionic surfactant is a polyoxyethylene derivative of a sorbitan fatty acid ester and preferably, a sorbitan mono fatty ester. More preferably, the sorbitan mono fatty ester surfactant is a polyoxyethylene derivative of a sorbitan fatty acid ester wherein the ester-forming fatty acid is selected from lauric acid, palmitic acid, oleic acid, and stearic acid. A nonionic surfactant having the desired chemical properties is Tween® 20 from Atlas Chemical Industries, Inc. Chemically, it is a polyoxyethylene 20 sorbitan monolaurate. Another suitable surfactant manufactured by Atlas is SPAN® 80 sorbitan monooleate.

Another suitable hydrophilic nonionic surfactant is one selected from a class of block copolymers of propylene oxide and ethylene oxide. One such water-soluble, nontoxic, nonionic surfactant is Poloxamer 407 (tradename Pluronic ® F127) from BASF Wyandotte Company. Its use is discussed in US-A-3 864 472 entitled Clear Lemon-flavored Mouthwash. Chemically the Pluronics® are block copolymers of propylene oxide and ethylene oxide containing various amounts of hydrophobe (polyoxypropylene) and hydrophile (polyoxyethylene). The Pluronic® F127 is characterized as a hydrophilic surfactant.

The surfactants and surfactant mixtures employed in this invention have a composite hydrophile-lipophile-balance (HLB) of between 9 and 30. Preferably, the sorbitan fatty acid ester is a polyoxyethylene derivative of sorbitan monolaurate having an HLB of 9 to 18, preferably 17, and the block copolymer of propylene oxide and ethylene oxide has an HLB of 15 to 30, preferably 22.

The block copolymer and the sorbitan fatty acid ester are preferably used in combination in a ratio of 2:1 to 200:1, and more preferably, in a ratio of 4:1 to 50:1. The present emulsions preferably contain 0.1 to 5 percent by weight sorbitan fatty acid ester and 0.1 to 20 percent by weight block copolymer of propylene oxide and ethylene oxide.

The emulsions of the present invention may also contain water-soluble, fluoride-containing, anticariogenic adjuvants. Preferably such an adjuvant is present in the form of water-soluble, fluoride-containing compounds capable of supplying fluoride ions. The preferred adjuvant is sodium fluoride, although other materials such as sodium monofluorophosphate, stannous fluorozirconate, indium fluorozirconate, stannous fluoride, and complex zirconium-germanium fluorides may be employed. Sodium fluoride is preferred by virtue of the absence of objectionable taste, lack of enamel pigmentation, the freedom from damage to gingival tissue, and by reason of anticariogenic effectiveness obtainable therewith. Other suitable adjuvants include water-soluble fluoride salts such as $NH_4F$, $SnF_4$, KF, $InF_3$, $PbF_2$, $FeF_2$, and LiF, as well as more complex water-soluble, fluoride-containing, adjuvants such as fluorosilicates, fluorozirconates, fluorostannites, fluoroborates, fluorotitanates, fluorogermanates, and mixed halides. Mixtures of suitable adjuvants may also be utilized. Aluminum fluoride may be used to supply both aluminum and fluoride to the system. In general, such fluoride adjuvants are present in anticariogenically effective and non-toxic amounts, typically at a level of about 0.05 up to 1.0 percent by weight of the dentifrice preparation so as to provide up to 1000 ppm fluoride ion.

The emulsions of this invention have demonstrated significant utility as anticariogenic agents for use in oral compositions comprising carriers such as water and other non-toxic materials. The compositions of this invention may be applied to teeth in aqueous solution of such carriers as in a topical treatment solution or in the form of a mouthwash. However, the compositions of the present invention are also well-suited for formulation of other oral compositions for dental caries prophylaxis, e.g., dentifrices and prophylaxis pastes/gels containing one or more compatible abrasives, lozenges, and chewing gums. Indeed, substantially any carrier capable of supplying active aluminum agent to the surface of the teeth may be employed in accordance with this invention.

In a preferred embodiment of the present invention, the emulsion is formed by mixing an aqueous solution of the aluminum salts with a sweetener and a blocked copolymer of propylene oxide and ethylene oxide. A second mixture of a polyoxyethylene derivative of a sorbitan fatty acid ester, flavor oil, and glycerin is prepared. The first and second mixtures are thereafter blended to form an emulsion of the present invention.

Emulsions of this invention are employed at their natural pH values, which range from 2.5 to 5.0 due to the Lewis acid effect of the aluminum. In a preferred embodiment, the pH of the emulsion ranges from 3.5 to 3.8. In all cases, the ingredients provided in the emulsions of this invention are selected so as to be compatible with aluminum ions.

Exemplary preparations employing the oral compositions of the present invention are given in the following Examples 1-5.

4

EP 0 270 977 B1

In Examples 2,3,4 and 5 the term "Sorbitol 70%" denotes a 70 weight percent solution of sorbitol in water.

| EXAMPLE 1 | |
|---|---|
| MOUTHWASH PREPARATION | |
| Ingredients | % By Weight |
| Distilled water | 85.070 |
| Pluronic® F127 (BASF Wyandotte) | 3.000 |
| $AlK(SO_4)_2 \cdot 12H_2O$ | 0.885 |
| Sodium saccharin | 0.100 |
| Glycerin | 10.000 |
| Tween® 20 (ICI Americas) | 0.600 |
| Flavor oil | 0.300 |
| 5% solution F D & C yellow dye #4 | 0.040 |
| 5% solution F D & C blue dye #1 | 0.005 |
| | 100.000% |

| EXAMPLE 2 | |
|---|---|
| DENTIFRICE PREPARATION | |
| Ingredients | % By Weight |
| Abrasive | 32.00 |
| Water | 20.31 |
| Glycerin | 17.50 |
| Sorbitol (70%) | 14.00 |
| NaOH (33 1/3%) | 1.00 |
| Binder | 1.00 |
| Pluronic®F-87 | 9.00 |
| $AlK(SO_4)_2 \cdot 12H_2O$ | 3.54 |
| Sodium saccharin | 0.25 |
| Tween®20 | 0.80 |
| Cassia flavor | 0.40 |
| Methyl paraben | 0.15 |
| Propyl paraben | 0.05 |
| | 100.00 |

| EXAMPLE 3 | |
|---|---|
| LOZENGE PREPARATION | |
| Ingredients | % By Weight |
| Sorbitol (70%) | 97.9 |
| Pluronic®F-127 | 1.2 |
| $AlCl_3 \cdot 6H_2O$ | 0.5 |
| Tween®80 | 0.2 |
| Flavorings, color | 0.2 |
| | 100.0 |

5

| EXAMPLE 4 | |
|---|---|
| CHEWING GUM PREPARATION | |
| Ingredients | % By Weight |
| Gum Base | 26.0 |
| Sorbitol powder | 47.9 |
| Sorbitol (70%) | 17.3 |
| Glycerin | 4.5 |
| Pluronic®F-127 | 2.0 |
| Tween®60 | 0.1 |
| $AlK(SO_4)_2 \cdot 12H_2O$ | 1.0 |
| Flavoring | 1.2 |
| | 100.0 |

| EXAMPLE 5 | |
|---|---|
| PROPHYLAXIS PASTE PREPARATION | |
| Ingredients | % By Weight |
| Abrasive | 48.0 |
| Water | 14.0 |
| Glycerin | 10.0 |
| Sorbitol (70%) | 6.0 |
| NaOH (33 1/3%) | 2.0 |
| Binders | 1.5 |
| Pluronic®- F88 | 8.0 |
| $Al(NO_3)_3 \cdot 9H_2O$ | 7.0 |
| Tween®80 | 1.0 |
| Sweeteners, flavor | 2.5 |
| | 100.0 |

Rat Dental Caries Study

The significant anticariogenic benefits of the aluminum-containing emulsion of this invention have been demonstrated in a dental caries study performed with rats.

The rat dental caries model used was that recommended by the National Institute of Dental Research NIH, (Larson, R.H. Et al., J. Dent. Res., 56:1007-1012, 1977). The study was designed to examine the effect of two experimental aluminum-containing dental rinses (Groups 3 and 4) on dental caries formation in the albino rat. The aluminum rinses were compared to a positive control fluoride rinse (Group 5) and two negative control cells consisting of water (Group 1) and a placebo rinse (Group 2). Furthermore, since aluminum has been demonstrated to enhance the effect of fluoride, a double treatment group (Group 6) was included in which the animals were treated first with aluminum followed by fluoride

Twenty litters of eight weanling Wistar strain rats were randomly distributed into eight equal groups of 20 animals according to sex, body weight, and litter mates. The rats were weighed initially, at one month, provided with NIDR cariogenic diet 2000 and distilled drinking water for the ten-week study.

All weanling rats initially were inoculated with the caries-inducing microorganisms, Streptococcus mutans 6715. The molars of the rats were swabbed with a 24-hour culture of the S. mutans 6715 and the residual was placed in their drinking water at a 1:100 dilution. The inoculations were repeated for 3 consecutive days to insure implantation of the microorganism.

The formulae for preparation of the dental rinse solutions used for treatment Groups 2-5 in the study are set forth in Table 1.

TABLE 1

| Dental Rinse Formulae for Rat Dental Caries Study | | | | |
|---|---|---|---|---|
| Ingredients | Percentage by weight | | | |
| | Group 2 Placebo | Group 3 AlK(SO$_4$)$_2$ | Group 4 AlCl$_3$ | Group 5 NaF |
| Distilled water | 85.955 | 84.177 | 85.062 | 85.800 |
| Pluronic® F127 | 3.000 | 3.000 | 3.00 | 3.000 |
| Aluminum potassium sulfate dodecahydrate | - | 1.758 | - | - |
| Aluminum chloride hexahydrate | - | - | 0.893 | - |
| Sodium fluoride | - | - | - | 0.155 |
| Sodium saccharin | 0.100 | 0.100 | 0.100 | 0.100 |
| Glycerin | 10.000 | 10.000 | 10.000 | 10.000 |
| Tween® | 0.600 | 0.600 | 0.600 | 0.600 |
| Citrus flavor | 0.300 | 0.300 | 0.300 | 0.300 |
| FD&C yellow dye #4 (5% solution) | 0.040 | 0.040 | 0.040 | 0.040 |
| FD&C blue dye #1 (5% solution) | 0.005 | 0.005 | 0.005 | 0.005 |
| Sodium hydroxide (33 1/3%) | - | q.s. | q.s. | - |
| Final pH | 6.5 | 3.8 | 3.8 | 6.5 |

The dental rinse solutions were prepared in accordance with the following procedure. The aluminum salt (or NaF) was first dissolved in 90% of the water with stirring. The sodium saccharin was then dissolved in the solution, following by the Pluronic® F127 surfactant. The solution was stirred for about one hour to totally dissolve the Pluronic® F127. The Tween® 20 and flavor oil were mixed in a separate container and the glycerin was subsequently added to and mixed with the flavor-oil Tween® 20 solution. The oil-surfactant mixture was slowly added to the aqueous mixture with stirring to form a clear emulsion. Coloring dyes were then added to the dental rinses. The pH of the aluminum rinses was slowly adjusted to 3.8 with the sodium hydroxide. The rinses were quantitatively transferred to a volumetric container of appropriate size, filled with the remaining distilled water, and used throughout the entire study.

Each hemijaw of each rat was swabbed for 15 seconds with the respective dental rinse twice daily, five days per week. The group treatment sequence was varied each day in order to minimize any potential error due to time of day of treatment.

After final weighing, the treated rats were sacrificed in pairs by chloroform inhalation and decapitated. The mandibles and maxillas were then surgically removed and defleshed in preparation for caries scoring. All animals were coded to prevent identification of the treatment vs. control groups by the examiner.

Dental caries was determined by scoring the number and severity of carious areas in sectioned teeth. The mandibles and maxillas were soaked overnight in an aqueous 0.05% ammonium purpurate (Murexide) solution to stain the decayed enamel. The buccal, lingual, and morsal surfaces were scored using a binocular dissection microscope and the location and size of lesions recorded on individual caries scoring charts. The hemijaws were subsequently sectioned mesially-distally into halves and graded for sulcal and proximal lesions.

The dental caries results are summarized in Table 2. This table includes the average final weight, smooth surface caries, and fissure caries scores, the percent reductions, and the statistical results. The smooth surface caries data, which are a combination of the buccal, lingual, and proximal lesion scores, represent the amount of dental decay observed on the outer surfaces of the rat teeth. The results demonstrated that the AlCl$_3$, AlK(SO$_4$)$_2$, and NaF rinses (Groups 3, 4, and 5, respectively) significantly reduced smooth surface dental caries by 43, 37, and 37%, respectively.

Numerically, the best reduction in smooth surface caries was observed for Group 6, which received the dual phase treatment of AlCl$_3$ and NaF. Although the 51% reduction observed for this double treatment group was significantly different from the controls, it was not statistically better than the 37% reductions observed for the individual NaF or AlCl$_3$ treatments

**TABLE 2**

**SUMMARY OF RAT DENTAL CARIES STUDY**

| Group | Treatment[a] | pH | n | Final Wt. (gm) Score[b] | Sig.[c] | Smooth Surface Caries Score[b] | Red.[c] | Sig.[c] | Fissure Caries Score[b] | Red.[c] | Sig.[c] |
|---|---|---|---|---|---|---|---|---|---|---|---|
| 1 | Distilled water | 6.5 | 20 | 239 | none | 11.10 | — | 3,4,5,6 | 24.45 | — | 3,4,5,6 |
| 2 | Placebo | 6.5 | 20 | 241 | none | 11.40 | — | 3,4,5,6 | 26.80 | — | 3,4,5,6 |
| 3 | AlK(SO$_4$)$_2$ | 3.8 | 20 | 241 | none | 6.45 | 43% | 1,2 | 17.35 | 35% | 1,2,5,6 |
| 4 | AlCl$_3$ | 3.8 | 20 | 246 | none | 7.20 | 37% | 1,2 | 17.35 | 35% | 1,2,5,6 |
| 5 | NaF | 6.5 | 20 | 237 | none | 7.15 | 37% | 1,2 | 7.45 | 72% | 1-4 |
| 6 | AlCl$_3$ & NaF | — | 20 | 242 | none | 5.55 | 51% | 1,2 | 6.75 | 75% | 1-4 |

a    All active agents present at a concentration of 0.037 Molar

b    Mean value, n = 20

c    The numbers represent the treatment groups which are significantly different according to the Newman-Keuls statistical test.

(Groups 4 and 5, respectively). However, these data seem to indicate that aluminum may enhance the effect of fluoride.

The fissure caries results also summarized in Table 2 are a combination of the sulcal and morsal lesions and represent the amount of dental decay observed in the pit and fissure areas of the rat molars. The results demonstrated that AlCl$_3$ and AlK(SO$_4$)$_2$ (Groups 3 and 4), significantly reduced the number of fissure caries by 35% each. NaF (Group 5) significantly reduced fissure caries by 72%. Group 6, treated

8

first with $AlCl_3$, and then NaF, resulted in the greatest reduction in fissure caries of 75%.

The overall results demonstrated that the ingredients used to formulate the dental rinse vehicle did not inactivate the aluminum or fluoride and did not possess any anticariogenic properties themselves. The rats in all six groups exhibited consistent and equivalent weight gains. This, coupled with the fact that no deaths occurred during the ten-week study period, provided further evidence regarding the lack of toxicity of aluminum. Thus, the dental rinse formulation was safe and effective to use as a vehicle for administering the aluminum in the human dental caries clinical study.

Human Dental Caries Clinical Investigation

A human clinical investigation was undertaken to determine the effectiveness of a topically-applied aluminum dental rinse prepared in accordance with the subject invention in reducing the incidence of dental caries compared with a positive control fluoride dentifrice. $AlK(SO_4)_2$ was used in the human dental caries study because of its GRAS (generally regarded as safe) status with the U.S. Food and Drug Administration.

A total of 260 elementary school children having a high incidence of caries was selected from three schools located in a low-fluoride area (0.4 ppm F or less in water supply). All children who were caries-free or had fissure sealants or orthodontic appliances were excluded. This precaution minimized the natural interference and variability these factors introduce into a dental caries clinical study. The children were examined both clinically and radiographically for dental caries and stratified into three groups. Group 1 served as the positive control and received a fluoridated dentifrice approved by the American Dental Association. Group 2 was given an experimental dental rinse containing 0.81% aluminum potassium sulfate with the pH adjusted to 3.8. In order the examine the anticariogenic effect of aluminum, it was essential that the subjects in Group 2 were not exposed to any exogenous sources of fluoride during the study. This necessitated: (1) conducting the study in an area where there was minimal fluoride content in the drinking water and (2) providing the children in Group 2 with a non-fluoride dentifrice. Group 3 received the same rinse as Group 2, but was supplied with the same fluoride dentifrice as Group 1 in order to examine the synergistic effect of aluminum and fluoride. For ethical reasons there was no placebo. The experimental groups were compared to the fluoride dentifrice positive group.

The dental rinses were self-administered under direct supervision at school after the noon meal, 5 days per week. When school was not in session, the rinses were self-applied at home under parental supervision. The procedure consisted of rinsing for 30 seconds each day with 10 ml of the dental rinse. After one month, the children were examined for gingivitis, mucosal irritations, enamel decalcification, etc., but no significant side effects were observed.

After six months, the children were again examined clinically and radiographically for dental caries. Because all groups exhibited a significant or directionaly positive effect at the six-month examination, the study was allowed to continue for a one-year examination period. At all times the double-blind status of the test was maintained. Examiner efficiency was maintained by scheduling reasonable numbers of examinations per session with adequate rest periods.

The dental rinses were as similar in appearance and taste as possible and were formulated in a form most palatable to children. The particular flavor of the rinses were changed periodically to provide variety and to maintain interest. Each batch was checked for enamel solubility reduction (ESR) in the laboratory before distribution in order to ensure cariostatic activity of the formulation.

The ESR tests were performed using the method described by Putt and Kleber (J. Dent. Res., 64:437-440, 1985).

Table 3 presents the ESR data for the six various flavored aluminum rinses utilized in the clinical study. Enamel dissolution by acid was reduced by approximately 70% after treatment with the aluminum dental rinses. The data indicated that all the aluminum-containing formulations were highly active and no significant incompatibilities occurred between aluminum and the other dental rinse ingredients. Placebo formulations without aluminum demonstrated no effect whatsoever in reducing enamel solubility, verifying that the aluminum was the active component.

Participants in Group 1 and 3 received adequate supplies of toothbrushes and a standard fluoride dentifrice (Crest®) repackaged in unmarked white tubes to use at home according to their normal oral hygiene habits. Participants in Group 2 were supplied in like manner with a standard non-fluoride dentifrice (Pepsodent®)

Examinations were performed by two dentists experienced in conducting dental caries studies. The techniques employed in the dental caries examinations were those recommended by the 1955 A.D.A. Dental Caries Symposium, the Ohio State Symposium, the 1961 Zurich Caries Symposium, and the 1968 A.D.A. Conference on the Clinical Testing of Cariostatic Agents.

During the examination procedures neither the examiners nor the trained recorder had knowledge of the

**TABLE 3**

**REDUCTION IN ENAMEL SOLUBILITY BY VARIOUS FLAVORED DENTAL RINSES USED IN HUMAN CLINICAL STUDY**

| Dental Rinse[a] | | | | Enamel Solubility Reduction Score[b] |
|---|---|---|---|---|
| Active Agent | Flavor | pH | n | |
| 0.885% AlK(SO$_4$)$_2$ ·12H$_2$O | 0.3% citrus-mint | 3.8 | 3 | 74 ± 3 |
| 0.885% AlK(SO$_4$)$_2$ ·12H$_2$O | 0.075% cassia | 3.8 | 6 | 71 ± 1 |
| 0.885% AlK(SO$_4$)$_2$ ·12H$_2$O | 0.3% lemon | 3.8 | 4 | 63 ± 2 |
| 0.885% AlK(SO$_4$)$_2$ ·12H$_2$O | 0.3% grape | 3.8 | 6 | 64 ± 3 |
| 0.885% AlK(SO$_4$)$_2$ ·12H$_2$O | 0.3% pineapple | 3.8 | 6 | 69 ± 4 |
| 0.885% AlK(SO$_4$)$_2$ ·12H$_2$O | 0.3% strawberry | 3.8 | 6 | 72 ± 3 |
| None | 0.075% cassia | 6.8 | 3 | -7 ± 5 |
| None | 0.3% lemon | 6.8 | 3 | -8 ± 9 |

a   All rinses prepared according to formula in Example 1, except for the pineapple flavor in which 1.2% Tween 20 was used.

b   Mean ± standard error

previous diagnosis or the group assignments. Two film, bite-wing radiographs were obtained immediately following the clinical examination. The radiographs were developed on-site and retakes obtained as needed.

The subjects were randomly assigned to the study groups after stratification based on dental caries susceptibility, dental age, and past dental caries experience. Control and test subjects were examined in random order. Only permanent teeth were included in the examination and diagnostic findings were recorded by teeth and surfaces. When lesions had been restored, they were recorded. Extracted teeth were recorded as were erupted noncarious teeth. Visual dental caries examination findings were called out in code to a trained recorder who wrote the information on an individual patient chart for each clinical examination.

The clinical dental caries findings were expressed in terms of the average increase in the number of decayed, missing, and filled teeth (DMFT) and surfaces (DMFS) per person in each group. The various components of each index, and the distribution on the various surfaces (occlusal, buccal-lingual, and proximal) were also obtained. A separate category was tabulated for teeth erupting during the study.

The six-month results for the aluminum rinse dental caries study are presented in Table 4. These results demonstrated clinically that aluminum is effective in reducing dental caries in humans.

The data in Table 4 demonstrate the differences (Δ) in decayed, missing, and filled teeth (DMFT) and

## TABLE 4

### DENTAL CARIES INCREMENTS AFTER SIX MONTHS

**Examiner A**

| Group | Treatment Rinse | Treatment Dentifrice | N | DMFT Mean | DMFT SEM[c] | DMFT % Red | DMFT Sig | DMFS Mean | DMFS SEM[c] | DMFS % Red | DMFS Sig |
|---|---|---|---|---|---|---|---|---|---|---|---|
| 1 | Placebo | Fluoride[a] | 77 | 1.00 | 0.162 | — | — | 1.62 | 0.268 | — | — |
| 2 | Aluminum[b] | Placebo | 80 | 0.58 | 0.168 | 42% | p=.04 | 1.04 | 0.265 | 36% | p=.06 |
| 3 | Aluminum[b] | Fluoride[a] | 80 | 0.59 | 0.140 | 41% | p=.03 | 0.89 | 0.215 | 45% | p=.02 |

**Examiner B**

| Group | Treatment Rinse | Treatment Dentifrice | N | DMFT Mean | DMFT SEM[c] | DMFT % Red | DMFT Sig | DMFS Mean | DMFS SEM[c] | DMFS % Red | DMFS Sig |
|---|---|---|---|---|---|---|---|---|---|---|---|
| 1 | Placebo | Fluoride[a] | 77 | 0.75 | 0.141 | — | — | 1.22 | 0.233 | — | — |
| 2 | Aluminum[b] | Placebo | 80 | 0.57 | 0.136 | 24% | p=.18 | 0.92 | 0.200 | 25% | p=0.16 |
| 3 | Aluminum[b] | Fluoride[a] | 80 | 0.67 | 0.154 | 10% | p=.35 | 0.97 | 0.220 | 21% | p=0.22 |

[a] Crest R With Fluoristat; contains 1100 ppm F as NaF

[b] Contains 500 ppm $Al^{+3}$ as $AlK(SO_4)_2 \cdot 12 H_2O$, pH 3.8

[c] Standard error of the mean

surfaces (DMFS) observed by both examiners after six months. The results for Examiner A demonstrated that: (10 a 30-second rinse with a 500 ppm aluminum solution five days per week reduced the DMFT and DMFS significantly better than daily use of a positive control fluoride dentifrice by 42% and 36%, respectively; and (2) the use of the aluminum rinse in combination with fluoride dentifrice resulted in similar statistically significant reductions in DMFT and DMFS of 41% and 45% respectively, compared to the fluoride dentifrice positive control group. The results of Examiner B likewise demonstrated that the two aluminum rinse groups reduced dental caries better than the fluoride positive control group.

11

The increments in DMFT and DMFS observed by both examiners after one year are presented in Table 5. The results confirmed the 6-month data that aluminum is effective in reducing dental caries in humans. For the most part, the results paralleled the findings for both examiners at six months. The results for Examiner A demonstrated that: (1) the 500 ppm aluminum dental rinse reduced $^\Delta$DMFT and $^\Delta$DMFS significantly better than daily use of a positive control fluoride dentifrice by 38% and 40% respectively; and (2) the use of aluminum rinse in combination with fluoride dentifrice resulted in similar statistically significant reductions in $^\Delta$DMFT and $^\Delta$DMFS of 32% and 32%, respectively, compared to the fluoride dentifrice positive control group. Thus, the significant caries reductions for aluminum observed by Examiner A at six months were still apparent after one year.

TABLE 5

DENTAL CARIES INCREMENTS AFTER ONE YEAR

Examiner A

| Group | Treatment Rinse | Dentifrice | N | DMFT Mean | SEM[c] | % Red | Sig | DMFS Mean | SEM[c] | % Red | Sig |
|---|---|---|---|---|---|---|---|---|---|---|---|
| 1 | Placebo | Fluoride[a] | 78 | 1.33 | 0.21 | -- | -- | 2.15 | 0.35 | -- | -- |
| 2 | Aluminum[b] | Placebo | 79 | 0.83 | 0.19 | 38% | p=.04 | 1.29 | 0.28 | 40% | p=.04 |
| 3 | Aluminum[b] | Fluoride[a] | 77 | 0.91 | 0.16 | 32% | p=.06 | 1.47 | 0.28 | 32% | p=.06 |

Examiner B

| Group | Treatment Rinse | Dentifrice | N | DMFT Mean | SEM[c] | % Red | Sig | DMFS Mean | SEM[c] | % Red | Sig |
|---|---|---|---|---|---|---|---|---|---|---|---|
| 1 | Placebo | Fluoride[a] | 78 | 1.12 | 0.17 | -- | -- | 1.90 | 0.26 | -- | -- |
| 2 | Aluminum[b] | Placebo | 79 | 1.04 | 0.17 | 7% | p=0.37 | 1.59 | 0.24 | 16% | p=0.19 |
| 3 | Aluminum[b] | Fluoride[a] | 77 | 1.06 | 0.19 | 5% | p=0.40 | 1.66 | 0.32 | 13% | p=0.28 |

[a] Crest® With Fluoristat; contains 1100 ppm F as NaF

[b] Contains 500 ppm $Al^{+3}$ as $AlK(SO_4)_2 \cdot 12H_2O$, pH 3.8

[c] Standard error of the mean

The results of Examiner B likewise demonstrated that the two aluminum rinse groups reduced dental caries better than the fluoride positive control group. However, all the percentage reductions were slightly less than Examiner A. This is believed to have resulted from the fact that Examiner B was not as critical as Examiner A in scoring incipient dental caries. The lack of additional cariostatic benefit for aluminum after one year was probably the result of the low decay rate during the second six-month period and the reduced usage of the aluminum rinses by the subjects due to unsupervised rinsing during the summer vacation period.

Table 6 lists the number of reversals in dental caries observed in each group for both examiners at the six-month and one-year examinations, which is indicative of remineralization or "healing" of incipient lesions. In all cases the two aluminum rinse groups results in a much greater number of reversals and of subjects with reversals than in the fluoride positive control. This was a very significant finding because it is known that fluoride is effective in remineralizing incipient lesions. Thus, reversals would be expected for the fluoride positive control. A greater percentage of the reversals was noted in the two aluminum groups for both examiners after both six and twelve months. For Examiner A, a greater percentage of subjects in the fluoride positive control group exhibited an increase in dental caries compared to the two aluminum groups.

Table 7 presents the number and percentage of subjects having caries-free permanent dentition during

## TABLE 6

## SUMMARY OF CHANGES IN DENTAL CARIES IN THE STUDY POPULATION

| | | | Percent Change in Dental Caries of Study Population | | | | | | | | | | | |
| | | | Six Months | | | | | | One Year | | | | | |
| | | | Examiner A | | | Examiner B | | | Examiner A | | | Examiner B | | |
| Group | Rinse | Dentifrice | Rev. | NC. | Inc. | Rev. | NC. | Inc. | Rev. | NC. | Inc. | Rev. | NC. | Inc. |
| 1 | Placebo | Fluoride | 12% | 26% | 62% | 12% | 38% | 51% | 10% | 24% | 65% | 4% | 36% | 60% |
| 2 | Aluminum | Placebo | 19% | 36% | 45% | 20% | 24% | 56% | 15% | 35% | 49% | 11% | 27% | 62% |
| 3 | Aluminum | Fluoride | 19% | 34% | 48% | 13% | 40% | 48% | 14% | 30% | 56% | 13% | 32% | 56% |

Rev. = Reversal in Dental Caries

NC. = No Change in Dental Caries

Inc. = Increase in Dental Caries

EP 0 270 977 B1

TABLE 7

NUMBER OF CARIES-FREE SUBJECTS OF EACH EXAMINATION

| Treatment | | | Caries-Free Subjects | | | | | | | | | | | |
|---|---|---|---|---|---|---|---|---|---|---|---|---|---|---|
| | | | Examiner A | | | | | | Examiner B | | | | | |
| | | | Initial | | 6-Month | | 1-Year | | Initial | | 6-Month | | 1-Year | |
| Group | Rinse | Dentifrice | N | (%) | N | (%) | N | (%) | N | (%) | N | (%) | N | (%) |
| 1 | Placebo | Fluoride | 10 | (12%) | 8 | (10%) | 6 | ( 8%) | 15 | (17%) | 8 | (10%) | 7 | ( 9%) |
| 2 | Aluminum | Placebo | 9 | (10%) | 7 | ( 9%) | 8 | (10%) | 14 | (16%) | 8 | (10%) | 8 | (10%) |
| 3 | Aluminum | Fluoride | 7 | ( 8%) | 5 | ( 6%) | 6 | ( 8%) | 11 | (13%) | 8 | (10%) | 9 | (12%) |

the course of the study. The number of subjects in the fluoride control group declined by one-half, while essentially no change was observed in the number of caries-free subjects in the two aluminum groups.

To investigate the possible cariostatic mechanism of aluminum, the clinical data for Examiner A were reanalyzed in three different ways: (1) the dental caries data for each group were broken down into subcategories with respect to tooth location and tooth surface type; (2) the new permanent teeth erupting during the study were examined for caries developing in each group; and (3) the reversal data for each group were subcategorized with respect to tooth location and surface.

This analysis indicated that the greatest caries protection afforded by the aluminum rinses occurred on

16

the buccal surfaces of the posterior teeth (although the overall decay rate for buccal surfaces was not that high compared to the occlusal and interproximal surfaces). Because the children held the aluminum rinse predominantly between their cheeks and the buccal surfaces of the posterior teeth while mouth rinsing, it is consistent that the buccal surfaces exhibited the greatest reduction in decay because of the longer contact time with the aluminum.

In general, the aluminum rinse provided its second greatest cariostatic effect on the occlusal surfaces compared to the fluoride positive control. Ordinarily fluoride provides the least effect on the occlusal surfaces. Consequently, administration of aluminum in a chewing gum vehicle may enable even more prolonged contact with the occlusal surfaces, thus providing a means of protecting an area that is both very susceptible to decay and generally unprotected by fluoride.

The aluminum rinse also provided a good effect interproximaly, but had a minimal effect on the lingual surfaces. However, the lingual surfaces likewise developed minimal dental caries.

Analysis of dental decay developing in the new permanent teeth that erupted during the course of the study demonstrated that very few of the newly erupted teeth in all three groups became carious. After one year only 10% of the newly erupted teeth in the fluoride positive control exhibited any decay on their surfaces. Even a lesser amount of decay (5% to 1%) was observed for the two aluminum groups. The predominant location for new carious development was the posterior occlusal surface. It appears that the aluminum-fluoride dual treatment may provide its greatest effect on newly erupting teeth.

Further analysis of the number and locations of reversals in DMFS occurring after six months and one year demonstrated that the majority of reversals occurred in the posterior dentition, predominantly with respect to the occlusal and interproximal surfaces. Moreover, the two aluminum rinse groups resulted in approximately twice as many reversals as fluoride in these two locations, especially in the interproximal locations. It therefore appears that aluminum provides greater remineralization of incipient lesions in those two surfaces most susceptible to dental decay.

The one-year dental caries clinical study demonstrated that aluminum in an acidic (pH 3.8) mouthwash emulsion, formed without use of stabilizing carboxylic acids, is a safe and effective agent in reducing the incidence of dental caries in humans. The study has given a strong indication that aluminum is actually superior to fluoride in cariostatic potential. Moreover, in this clinical study the odds were in favor of the Crest® dentifrice fluoride positive control. It was probably used more than the aluminum rinse (daily versus 4-5 times per week), it contained more cariostatic agent (1100 ppm F versus 500 ppm $Al^{+3}$), and it has been clinically proven to prevent dental decay by 40%, compared to the approximately 25% reductions noted for most other commercial fluoride dentifrices.

Surfactant Compatibility Study

Representative samples of surfactants from common chemical subclasses within each class (anionic, nonionic, cationic, and amphoteric) of surfactants were selected and examined for their effect on enamel solubility reduction (ESR) using the procedure described by Applicants in J. Dent. Res., 64:437-440 (1985). Results from these studies are presented in Tables 8 and 9.

## TABLE 8

### ESR, pH, AND VISUAL APPEARANCE OF
### ANIONIC, NONIONIC, CATIONIC, AND AMPHOTERIC SURFACTANTS

| TYPE OF SURFACTANT | EXAMPLE TESTED | TRADE NAME | SOURCE | SURFACTANT 1% CONC. pH SOL. | CALCIUM ESR 1 PTD | | 4 PTD | | PHOSPHORUS ESR 1 PTD | | 4 PTD | |
|---|---|---|---|---|---|---|---|---|---|---|---|---|
| | | | | | MN | SD | MN | SD | MN | SD | MN | SD |
| CONTROL | distilled water | none | stock | 6.6 tr | 2 | 7 | -6 | 6 | 14 | 17 | -2 | 18 |
| I.ANIONIC | | | | | | | | | | | | |
| A.Carboxylates | | | | | | | | | | | | |
| 1.regular | sodium stearate | none | stock | 10.7 cl,fm | 10 | 7 | -17 | 8 | 13 | 9 | -19 | 9 |
| 2.polyalkoxycarboxylates | | Sandopan MS-40 | Sandoz | 6.1 tr | 8 | 8 | -4 | 1 | 2 | 2 | -4 | 1 |
| B.N-Acylsarcosinates | Na lauryl sarcosine | Hamposyl L | WR Grace | 3.3 i,ppt | 26 | 8 | 8 | 8 | 20 | 10 | 5 | 4 |
| | Na lauryl sarcosinate | Hamposyl L.30 | WR Grace | 6.6 tr | 12 | 10 | -25 | 14 | 4 | 10 | -27 | 7 |
| C.Acylated Protein Hydrolysates | none | none | none | | | | | | | | | |
| D.Sulfonates | | | | | | | | | | | | |
| 1.alkylbenzenesulfonates | | Conco AAS-45S | Con Chem | | | | | | | | | |
| 2.alkylarenesulfonates | | | | | | | | | | | | |
| 3.lignosulfonates | | | | | | | | | | | | |
| 4.naphthalenesulfonates | | Daxad 11 | WR Grace | 6.6 tr | -4 | 2 | 2 | 10 | -7 | 2 | -1 | 8 |
| 5.a-olefinsulfonates | | Conco AOS-40 | Con Chem | | | | | | | | | |
| 6.petroleum sulfonates | | | | | | | | | | | | |
| 7.dialkylsultosuccinates | | Aerosol OT | Am Cyn | 5.7 tr | -4 | 7 | -3 | 14 | -6 | 10 | -7 | 13 |
| 8.amidosulfonates | | Concogel | Con Chem | | | | | | | | | |
| 9.acyl isethionates | | Dowfax 2A1 | Dow Chem | 7.5 tr | 1 | 2 | -8 | 3 | -2 | 3 | -11 | 1 |
| E.Sulfates and Sulfonated Products | | | | | | | | | | | | |
| 1.alcohol sulfates | Na lauryl sulfate | | stock | 8.0 tr,fm | -3 | 14 | -18 | 10 | -3 | 10 | -21 | 12 |
| 2.ethoxylated alcohol sulfates | | Conco Sulfate -219 | Con Chem | | | | | | | | | |
| 3.ethoxylated alkylphenol sulfates | | Triton X-301 | Rohm/Haas | 6.7 tr | -1 | 6 | -10 | 9 | 1 | 4 | -13 | 7 |
| 4.sulfated acids,amides, & esters | | | | | | | | | | | | |
| 5.sulfated oils & fats | | | | | | | | | | | | |
| F.Phosphate Esters | Na nonoxynol-6 phosphate | Emphos CS-1361 | Witco | | | | | | | | | |
| II.NONIONIC | | | | | | | | | | | | |
| A.Ethoxylates | | | | | | | | | | | | |
| 1.alcohol ethoxylates | | Brig 35 | ICI Amer | 3.6 tr | 17 | 1 | -6 | 13 | 21 | 1 | -5 | 13 |
| | alkylated polyether alcohol | Triton X-100 | Rohm/Haas | 4.1 tr,fm | 8 | 7 | -5 | 12 | 7 | 6 | -5 | 14 |
| 2.alkylphenol ethoxylates | | Igepal CO-660 | GAF Co | 6.6 tr | 26 | 1 | -3 | 6 | 24 | 1 | -7 | 10 |
| B.Carboxylic (Fatty) Acid Esters | | | | | | | | | | | | |
| 1.glycerol esters | glycerol monostearate | Adacel 165 | ICI Amer | | | | | | | | | |
| | glycerol & polyoxy- ethylene stearate | Arlacel 165 | ICI Amer | 4.7 v cl | -5 | 7 | -15 | 9 | -7 | 7 | -18 | 7 |
| 2.polyoxyethylene esters | | Myrj 52S | ICI Amer | 6.1 tr | -1 | 6 | -2 | 4 | -2 | 6 | -7 | 5 |
| | polyoxyethylene 20 isohexadecyl ether | Arlasolve 200 | ICI Amer | 4.1 tr | 2 | 8 | -6 | 8 | 1 | 8 | -7 | 7 |
| 3.anhydrosorbitol esters | | Span 20 | ICI Amer | 5.7 cl | -3 | 5 | -14 | 9 | -3 | 4 | -13 | 6 |
| | | Span 40 | ICI Amer | | | | | | | | | |
| | | Span 60 | ICI Amer | 7.3 cl | 0 | 3 | 0 | 2 | 2 | 6 | -3 | 2 |

**TABLE 8 (continued)**

| TYPE OF SURFACTANT | EXAMPLE TESTED | TRADE NAME | SOURCE | SURFACTANT 1% CONC. pH | SOL. | CALCIUM ESR 1 PTD MN | SD | 4 PTD MN | SD | PHOSPHORUS ESR 1 PTD MN | SD | 4 PTD MN | SD |
|---|---|---|---|---|---|---|---|---|---|---|---|---|---|
| 4. ethoxylated anhydrosorbitol esters |  | Tween 20 | ICI Amer | 4.8 | tr | 17 | 2 | -8 | 9 | 17 | 4 | -13 | 9 |
|  |  | Tween 60 | ICI Amer | 4.4 | s.cl | 15 | 6 | -4 | 1 | 15 | 7 | -6 | 2 |
|  |  | Tween 80 | ICI Amer |  |  |  |  |  |  |  |  |  |  |
| 5. ethoxylated fats, oils, & waxes | ethoxylated castor oil | Emulphor EL719 | GAF Co | 6.6 | tr | 1 | 12 | -16 | 10 | 4 | 8 | -11 | 6 |
|  | polyoxyethylene sorbitol oleate-laurate | Atlox 1045A | ICI Amer | 7.0 | v.cl | -1 | 7 | -2 | 6 | 6 | 17 | 1 | 7 |
|  | polyoxyethylene sorbitol lanolin | G-1441 | ICI Amer | 7.6 | s.cl | 8 | 7 | -12 | 9 | 4 | 12 | -11 | 10 |
|  | diethylene glycol distearate | | stock | 6.1 | i | 7 | 2 | -8 | 8 | 8 | 3 | -8 | 10 |
| 6. glycerol esters of fatty acids |  |  |  |  |  |  |  |  |  |  |  |  |  |
| C. Carboxylic Amides |  |  |  |  |  |  |  |  |  |  |  |  |  |
| 1. diethanolamine condensates |  | Condensate PO | Con Chem |  |  |  |  |  |  |  |  |  |  |
| 2. monoalkanolamine condensates |  |  |  |  |  |  |  |  |  |  |  |  |  |
| 3. polyoxyethylene fatty acid amides | PEG-6-lauramide | Amidox L-5 | Stepan | 9.5 | tr | 0 | 4 | -3 | 8 | -1 | 5 | -2 | 7 |
| D. Polyalkene Oxide Block Copolymers |  |  |  |  |  |  |  |  |  |  |  |  |  |
| 1. poly(oxyethylene-co-oxypropylene) |  | Pluronic F127 | ICI Amer | 5.6 | tr,fm | -3 | 11 | -7 | 0 | -6 | 13 | -13 | 3 |
|  |  | Pluronic F87 | ICI Amer | 6.1 | tr,fm | 21 | 1 | -3 | 4 | 21 | 3 | -7 | 2 |
| III. CATIONIC A. Amines |  |  |  |  |  |  |  |  |  |  |  |  |  |
| 1. oxygen-free amines |  |  |  |  |  |  |  |  |  |  |  |  |  |
| 2. amine oxides | lauramine oxide | Ammonyx LO | Stepan | 7.5 | tr,fm | 26 | 3 | -8 | 9 | 24 | 2 | -8 | 7 |
| 3. alkylamine ethoxylates |  | Triton RW-75 | Rohm/Haas | 10.8 | tr | -6 | 8 | -11 | 6 | -7 | 5 | -16 | 2 |
| 4. ethylenediamine alkoxylates |  | Tetronic Polyol-704 | BASF Wyn | 9.0 | tr | -11 | 10 | -2 | 3 | -16 | 5 | -9 | 5 |
| 5. amines with amide linkages | coco dimethlammonium salt | Andogen 464 | Aldrich | 3.9 | cl,fm | 9 | 7 | -11 | 3 | 13 | 5 | -9 | 2 |
| B. Quarternary Ammonium Salts | quarternary ammonium derivative | G-3634A | ICI Amer | 5.0 | tr | 1 | 9 | -8 | 0 | -2 | 9 | -8 | 7 |
|  |  | G-263 | ICI Amer | 3.9 | tr | 0 | 1 | -7 | 3 | -3 | 1 | -11 | 5 |
| IV. AMPHOTERIC A. Amino Acids | glycine |  |  | 6.2 | tr | 3 | 6 | 0 | 14 | 5 | 7 | -3 | 17 |
|  | cysteine |  |  | 4.4 | tr | 6 | 13 | 6 | 9 | 7 | 12 | 7 | 6 |
|  | arginine |  |  | 11.0 | tr | -6 | 10 | -17 | 24 | 8 | 9 | -2 | 19 |
|  | aspartic |  |  | 2.9 | tr | -16 | 5 | -15 | 12 | -8 | 5 | -13 | 15 |
|  | phenylalanine |  |  | 5.6 | tr | 14 | 12 | 0 | 19 | 13 | 8 | 0 | 18 |
| B. Imidazolinium Derivatives | alkyl betaine | Emcol CC37-18 | Witco |  |  |  |  |  |  |  |  |  |  |

Abbreviations: tr = transparent or clear, cl = cloudy, v = very, s = slightly i = insoluble, fm = foamy, ppt = precipitate,

Table 8 presents the data for the surfactant samples tested without aluminum present. The table lists the chemical class name of the surfactant, the example tested, its corresponding trade name and manufacturer, if appropriate, the pH and visible appearance of a 1% test concentration, and the resulting enamel solubility reduction (ESR) scores. ESR scores represent the percent degree to which the surfactant treatment was able to reduce the dissolution of tooth enamel in acid, both immediately after treatment (1 PTD scores) and following four additional acid demineralizations (4 PTD scores). The higher the score, the better the efficiency of the treatment solution.

In addition to calculating the ESR scores from the amount of enamel calcium found in the demineralization solutions, enamel phosphorous was also measured as a check. Although essentially equivalent, both the calcium and phosphorous ESR scores are presented. Three replicates were conducted in order to establish the mean (MN) and standard deviation (SD) values. Comparing the ESR data in Table 8 to the distilled water control, it is apparent that none of the surfactants by themselves had any significant effect in reducing the acid solubility of enamel. The slight effect noted for Hamposyl® L, Igepal® CO-660, Pluronic® F87, and Ammonyx® LO was probably due to a slight coating of the tooth surface by the surfactant. Indeed the 4 PTD scores show that this effect was totally transient with no residual benefit. Most of the surfactant resulted in transparent (tr) aqueous solutions, although some produced cloudy (cl) and even insoluble (i) mixtures.

The corresponding pH, visual compatibility, and ESR data for the same surfactants combined with 0.005 M AlK(SO₄)₂•12H₂O are presented in Table 9. A low concentration of 0.005 M aluminum was used so that any minor interference caused by the surfactants would be more readily detected by the ESR procedure. From the data, it is apparent that, compared to the aluminum positive control, the anionic surfactant types generally are not compatible with aluminum and significantly diminish its ability to reduce the acid dissolution of enamel. This is not totally unexpected since the negative charge of such anionic surfactants can combine with the positively charged aluminum cations to inactivate them. Interestingly, the Sandopan® MS-40 anionic surfactant is an exception. Sandopan® MS-40, a polyethoxycarboxylate, had excellent ESR scores, and could be used in the present aluminum-containing anticariogenic systems. The anionic Triton® X-301, an ethoxylated alkylphenol sulfate, also gave an acceptable ESR.

The uncharged, nonionic surfactants resulted in ESR scores indicative of good compatibility with aluminum. All of the positively charged cationic amine surfactants, except for Tetronic Polyol® 704 were incompatible with aluminum. Apparently polyalkoxylation of the ethylenediamine moiety results in a predominant aluminum compatible nonionic-like character comparable to the character described for the

TABLE 9

ESR, pH, AND VISUAL APPEARANCE OF 0.005M ALUMINUM WITH ANIONIC, NONIONIC, CATIONIC, AND AMPHOTERIC SURFACTANTS

| TYPE OF SURFACTANT | EXAMPLE TESTED | TRADE NAME | SOURCE | .005M ALUM + 1% SURFACTANT pH SOL. | CALCIUM ESR 1 PTD MN | SD | 4 PTD MN | SD | PHOSPHORUS ESR 1 PTD MN | SD | 4 PTD MN | SD |
|---|---|---|---|---|---|---|---|---|---|---|---|---|
| CONTROL | AlK(SO4)2-12H20 | alum | Baker | 4.0 tr | 80 | 3 | 59 | 5 | 81 | 3 | 61 | 6 |
| I. ANIONIC | | | | | | | | | | | | |
| A. Carboxylates | | | | | | | | | | | | |
| 1. regular | sodium stearate | none | stock | 9.4 ppt,v cl | 47 | 11 | 23 | 5 | 50 | 1 | 23 | 4 |
| 2. polyalkoxycarboxylates | | Sandopan MS-40 | Sandoz | 3.9 s cl | 69 | 5 | 31 | 14 | 70 | 3 | 34 | 12 |
| B. N-Acylsarcosinates | Na lauryl sarcosine | Hamposyl L | WR Grace | 2.5 i.ppt | 57 | 12 | 28 | 2 | 57 | 1 | 31 | 3 |
| | Na lauryl sarcosinate | Hamposyl L-30 | WR Grace | 3.4 ppt,cl | 43 | 4 | 20 | 7 | 46 | 4 | 21 | 9 |
| C. Acylated Protein Hydrolysates | none | none | | | | | | | | | | |
| D. Sulfonates | | | | | | | | | | | | |
| 1. alkylbenzenesulfonates | | Conco AAS-45S | Con Chem | | | | | | | | | |
| 2. alkylarenesulfonates | | | | | | | | | | | | |
| 3. lignosulfonates | | | | | | | | | | | | |
| 4. naphthalenesulfonates | | Darad 11 | WR Grace | 4.2 ppt,cl | 56 | 2 | 37 | 8 | 56 | 1 | 36 | 8 |
| 5. a-olefinsulfonates | | Conco AOS-40 | Con Chem | | | | | | | | | |
| 6. petroleum sulfonates | | | | | | | | | | | | |
| 7. dialkylsulfosuccinates | | Aerosol OT | Am Cyn | 4.1 ppt,cl | 27 | 7 | -12 | 16 | 27 | 2 | -9 | 13 |
| 8. amidosulfonates | | Concogel | Con Chem | | | | | | | | | |
| 9. acyl isethionates | | Dowfax 2A1 | Dow Chem | 4.1 ppt,s cl | 69 | 4 | 31 | 10 | 66 | 11 | 28 | 9 |
| E. Sulfates and Sulfonated Products | | | | | | | | | | | | |
| 1. alcohol sulfates | Na lauryl sulfate | Conco Sulfate -219 | stock | 4.2 ppt,fm | 56 | 4 | 27 | 10 | 56 | 2 | 29 | 12 |
| 2. ethoxylated alcohol sulfates | | | Con Chem | | | | | | | | | |
| 3. ethoxylated alkylphenol sulfates | | Triton X-301 | Rohm/Haas | 3.9 ppt,s cl | 73 | 9 | 42 | 4 | 68 | 10 | 40 | 4 |
| 4. sulfated acids, amides, & esters | | | | | | | | | | | | |
| 5. sulfated oils & fats | | | | | | | | | | | | |
| F. Phosphate Esters | Na nonoxynol-6 phosphate | Emphos CS-1361 | Witco | | | | | | | | | |
| II. NONIONIC | | | | | | | | | | | | |
| A. Ethoxylates | | | | | | | | | | | | |
| 1. alcohol ethoxylates | alkylated polyether alcohol | Brig 35 | ICI Amer | 3.8 tr | 81 | 2 | 61 | 6 | 82 | 2 | 64 | 4 |
| | | Triton X-100 | Rohm/Haas | 3.7 tr,s ppt | 76 | 10 | 66 | 15 | 79 | 9 | 65 | 14 |
| 2. alkylphenol ethoxylates | | Igepal CO-660 | GAF Co | 4.0 tr | 78 | 4 | 65 | 7 | 80 | 4 | 61 | 7 |
| B. Carboxylic (Fatty) Acid Esters | | | | | | | | | | | | |
| 1. glycerol esters | glycerol monostearate | Adacel 165 | ICI Amer | 3.9 v cl | 80 | 8 | 48 | 10 | 79 | 6 | 46 | 7 |
| | glycerol & polyoxy-ethylene stearate | Arlacel 165 | ICI Amer | | | | | | | | | |
| 2. polyoxyethylene esters | polyoxyethylene 20 isohexadecyl ether | Myrj 52S | ICI Amer | 3.8 tr | 74 | 3 | 39 | 8 | 75 | 3 | 39 | 8 |
| | | Arlasolve 200 | ICI Amer | 3.9 tr | 80 | 6 | 58 | 5 | 81 | 5 | 57 | 5 |
| 3. anhydrosorbitol esters | | Span 20 | ICI Amer | 3.9 cl | 75 | 6 | 44 | 6 | 79 | 5 | 47 | 9 |
| | | Span 40 | ICI Amer | 4.1 cl | 72 | 9 | 37 | 6 | 73 | 7 | 40 | 7 |
| | | Span 60 | ICI Amer | | | | | | | | | |

TABLE 9
(continued)

| TYPE OF SURFACTANT | EXAMPLE TESTED | TRADE NAME | SOURCE | .005M ALUM + 1% SURFACTANT pH | SOL. | CALCIUM ESR 1 PTD MN | SD | 4 PTD MN | SD | PHOSPHORUS ESR 1 PTD MN | SD | 4 PTD MN | SD |
|---|---|---|---|---|---|---|---|---|---|---|---|---|---|
| 4.ethoxylated anhydrosorbitol esters | | Tween 20 | ICI Amer | 3.8 | tr | 73 | 2 | 50 | 9 | 77 | 1 | 50 | 10 |
| | | Tween 60 | ICI Amer | 3.8 | cl | 78 | 3 | 66 | 7 | 81 | 3 | 65 | 8 |
| | | Tween 80 | ICI Amer | | | | | | | | | | |
| 5.ethoxylated fats,oils, & waxes | ethoxylated castor oil | Emulphor EL719 | GAF Co | 3.9 | s cl | 73 | 4 | 35 | 2 | 75 | 4 | 35 | 1 |
| | polyoxyethylene sorbitol oleate-laurate | Atlox 1045A | ICI Amer | 4.0 | v cl | 79 | 2 | 44 | 4 | 82 | 2 | 49 | 2 |
| 6.glycerol esters of fatty acids | polyoxyethylene sorbitol lanolin diethylene glycol disterate | G-1441 | ICI Amer | 4.0 | s cl | 81 | 5 | 47 | 13 | 81 | 6 | 43 | 11 |
| | | | stock. | 3.5 | i | 74 | 7 | 39 | 5 | 75 | 6 | 40 | 7 |
| C.Carboxylic Amides | | Condensate PO | Con Chem | | | | | | | | | | |
| 1.diethanolamine condensates | | | | | | | | | | | | | |
| 2.monoalkanolamine condensates | | | | | | | | | | | | | |
| 3.polyoxyethylene fatty acid amides | PEG-6-lauramide | Amidox L-5 | Stepan | 4.2 | cl | 67 | 16 | 38 | 4 | 67 | 15 | 39 | 3 |
| D.Polyalkene Oxide Block Copolymers | | Pluronic F127 | ICI Amer | 3.8 | tr,fm | 77 | 5 | 52 | 17 | 76 | 4 | 49 | 11 |
| 1.poly(oxyethylene-co-oxypropylene) | | Pluronic F87 | ICI Amer | 3.8 | tr,fm | 81 | 2 | 67 | 2 | 79 | 2 | 61 | 5 |
| III.CATIONIC | | | | | | | | | | | | | |
| A.Amines | | | | | | | | | | | | | |
| 1.oxygen-free amines | | Ammonyx LO | Stepan | 4.3 | ppt,cl | 36 | 9 | 21 | 8 | 38 | 10 | 24 | 6 |
| 2.amine oxides | | Triton RW-75 | Rohm/Haas | 8.8 | s cl | 1 | 9 | -20 | 8 | 4 | 8 | -23 | 11 |
| 3.alkylamine ethoxylates | | Tetronic Polyol-704 | BASF Wyn | 4.0 | s cl | 82 | 6 | 53 | 8 | 81 | 4 | 47 | 5 |
| 4.ethylenediamine alkoxylates | lauramine oxide | Andogen 464 | Aldrich | 3.5 | cl,fm | 80 | 8 | 52 | 17 | 77 | 8 | 51 | 17 |
| 5.amines with amide linkages | | | | | | | | | | | | | |
| B.Quarternary Ammonium Salts | coco dimethlammonium salt | G-3634A | ICI Amer | 4.0 | tr | 77 | 3 | 38 | 6 | 75 | 2 | 36 | 5 |
| | quarternary ammonium derivative | G-263 | ICI Amer | 3.9 | tr | 81 | 3 | 61 | 2 | 83 | 2 | 61 | 2 |
| IV.AMPHOTERIC | | | | | | | | | | | | | |
| A.Amino Acids | glycine | | | 4.0 | cl | 50 | 3 | 30 | 7 | 54 | 3 | 34 | 9 |
| | cysteine | | | 2.9 | tr | 71 | 2 | 40 | 9 | 74 | 1 | 42 | 10 |
| | arginine | | | 9.8 | cl | 7 | 10 | -24 | 22 | 17 | 1 | -16 | 24 |
| | aspartic | | | 2.9 | tr | 40 | 8 | 18 | 7 | 37 | 9 | 18 | 6 |
| | phenylalanine | | | 3.7 | s cl | 70 | 9 | 48 | 9 | 68 | 7 | 38 | 8 |
| B.Imidazolinium Derivatives | alkyl betaine | Emcol CC37-18 | Witco | | | | | | | | | | |

Abbreviations: tr = transparent or clear, cl = cloudy, i = insoluble, fm = foamy, ppt = precipitate, v = very, s = slightly

polyalkoxycarboxylates (Sandopan® MS-40) above. The incompatibility of amines with aluminum in aqueous solution is due to the relatively high pH of the amines and/or formation of an insoluble aluminum amide salt. The cationic quaternary ammonium salts are, however, functional with aluminum. Regarding the dual charged amphoteric surfactants, those derived from amino acids of glycine, cysteine, and phenylalanine resulted in acceptable ESR scores. Surfactants derived from arginine and aspartic acid provided low ESR scores, presumably because of their inherent high and low pH, respectively.

Based on the results of surfactant/aluminum compatibility tests, compatible surfactant blends can also be utilized to obtain the optimum HLB (hydrophilic-lipophilic balance) required to meet the needs of any particular aluminum emulsion system.

**Claims**

1. A palatable anticariogenic emulsion adapted for application to teeth, said emulsion having a pH of 2.5 to 5.0 and comprising

    10 to 50 000 ppm aluminum ions capable of reacting with the teeth,
    a sweetener,

21

0.1 to 5.0 weight percent of a flavour oil,

water, and

hydrophilic nonionic surfactants comprising (1) 0.1 to 5 percent by weight polyoxyethylene derivatives of sorbitan fatty acid esters and (2) 0.1 to 20 percent by weight block copolymers of propylene oxide and ethylene oxide, said surfactants being present in an amount effective to stabilize the anticariogenic emulsion.

2. The anticariogenic emulsion of claim 1 in the form of a product selected from the group consisting of a dental rinse, a mouthwash, a toothpaste, a prophylaxis paste, a chewing gum, and a lozenge.

3. The emulsion of claim 1 wherein the aluminum ions are derived from a water-soluble aluminum salt selected from the group consisting of aluminum potassium sulfate, aluminum chloride, aluminum sodium sulfate, aluminum sodium phosphate, aluminum sulfate, and aluminum nitrate.

4. The emulsion of claim 1 further comprising an ingredient selected from the groups consisting of 1 to 90 weight percent of a humectant selected from glycerine and sugar alcohols, and wherein the aluminum ions are present at a level of 250 to 10 000 ppm.

5. The emulsion of claim 4 wherein the humectant is selected from the group consisting of glycerin, xylitol, mannitol and sorbitol.

6. The emulsion of claim 4 wherein the sweetener comprises a non-cariogenic sweetener selected from the group consisting of saccharin, cyclamate and aspartame.

7. The emulsion of claim 4 wherein the hydrophilic nonionic surfactants are a polyoxyethylene derivative of a sorbitan mono fatty ester and a block copolymer of propylene oxide and ethylene oxide having an HLB of between 9 and 30.

8. The emulsion of claim 4 wherein the sorbitan mono fatty ester surfactant is a polyoxyethylene derivative of a sorbitan fatty acid ester wherein the ester-forming fatty acid is selected from the group consisting of lauric acid, palmitic acid, oleic acid, and stearic acid.

9. The emulsion of claim 8 wherein the sorbitan fatty acid ester surfactant and the block copolymer are present in a weight ratio of 1:2 to 1:200.

10. The emulsion of claim 9 having a pH of 3.5 to 3.9 with aluminum ions at 500 ppm.

11. The emulsion of claim 10 wherein the sorbitan fatty acid ester is a polyoxyethylene derivative of sorbitan monolaurate having an HLB of 9 to 18 and the block copolymer of propylene oxide and ethylene oxide has an HLB of 15 to 30.

12. The emulsion of claim 4 wherein the aluminum ions are present at 500 to 2000 ppm and the sorbitan fatty acid ester is a polyoxyethylene derivative of sorbitan monolaurate having an HLB of 17 and the blocked copolymer of propylene oxide and ethylene oxide has an HLB of 22.

13. The emulsion of claim 12 in the form of a dental rinse or mouthwash.

14. The product of claim 13 wherein the sorbitan fatty acid ester constitutes 0.1 to 5 percent by weight of the product and the block copolymer of propylene oxide and ethylene oxide constitutes 1 to 20 percent weight of the product.

15. The product of claim 14 wherein the polyoxyethylene derivative of a sorbitan monolaurate and the block copolymer of propylene oxide and ethylene oxide have an HLB of 17 and 22 respectively and are used in a weight ratio of 1:4 to 1:50.

16. The emulsion of claim 1 containing in addition, water-soluble fluoride-containing salts.

17. The emulsion of claim 12 containing in addition 100 to 1000 ppm fluoride ions.

**18.** A palatable anticariogenic emulsion adapted for application to teeth, said emulsion having a pH of 2.5 to 5.0 and comprising

10 to 50 000 ppm aluminum ions capable of reacting with the teeth,

a sweetener,

0.1 to 5.0 weight percent of a flavour oil,

water, and

a surfactant selected from the group consisting of polyalkoxylated anionic surfactants, cationic polyalkoxyamines, quaternary ammonium salts, and amphoteric surfactants derived from amino acids selected from the group consisting of glycine, cysteine, and phenylalanine, said surfactants being present in an amount effective to stabilize the anticariogenic emulsion.

**19.** The anticariogenic emulsion of claim 18 in the form of a product selected from the group consisting of a dental rinse, a mouthwash, a toothpaste, a prophylaxis paste, a chewing gum, and a lozenge.

**20.** The emulsion of claim 18 further comprising an ingredient selected from the group consisting of 1 to 90 weight percent of a humectant selected from glycerin and sugar alcohols, and wherein the aluminum ions are present at a level of 250 to 10 000 ppm.

**21.** The emulsion of claim 20 wherein the humectant is selected from the group consisting of glycerin, xylitol, mannitol and sorbitol.

**22.** The emulsion of claim 20 wherein in the sweetener comprises a non-cariogenic sweetener selected from the group consisting of saccharin, cyclamate and aspartame.

**23.** The emulsion of claim 20 wherein the surfactant as an HLB of between 9 and 30.

**24.** The emulsion of claim 23 wherein the surfactant is selected from the group consisting of polyalkoxylated anionic and polyalkoxylated cationic surfactants.

**25.** The emulsion of claim 23 wherein the surfactant is a quaternary ammonium salt.

**26.** The emulsion of claim 23 wherein the surfactant is a derivative of an amino acid selected from the group consisting of glycine, cysteine and phenylalanine.

**Claims for the following Contracting State : ES**

**1.** A process for preparing a palatable anticariogenic emulsion adapted for application to teeth, said emulsion having a pH of 2.5 to 5.0, **characterized** by mixing

10 to 50 000 ppm aluminum ions capable of reacting with the teeth,

a sweetener,

0.1 to 5.0 weight percent of a flavour oil,

water, and

hydrophilic nonionic surfactants comprising (1) 0.1 to 5 percent by weight polyoxyethylene derivatives of sorbitan fatty acid esters and (2) 0.1 to 20 percent by weight block copolymers of propylene oxide and ethylene oxide, said surfactants being present in an amount effective to stabilize the anticariogenic emulsion.

**2.** The process of claim 1, **characterized** in that a product selected from the group consisting of a dental rinse, a mouthwash, a toothpaste, a prophylaxis paste, a chewing gum, and a lozenge is prepared.

**3.** The process of claim 1, **characterized** in that the aluminum ions are derived from a water-soluble aluminum salt selected from the group consisting of aluminum potassium sulfate, aluminum chloride, aluminum sodium sulfate, aluminum sodium phosphate, aluminum sulfate, and aluminum nitrate.

**4.** The process of claim 1, **characterized** by adding to the mixture an ingredient selected from the groups consisting of 1 to 90 weight percent of a humectant selected from glycerine and sugar alcohols, and wherein the aluminum ions are present at a level of 250 to 10 000 ppm.

23

5. The process of claim 4, **characterized** in that the humectant is selected from the group consisting of glycerin, xylitol, mannitol and sorbitol.

6. The process of claim 4, **characterized** in that the sweetener comprises a non-cariogenic sweetener selected from the group consisting of saccharin, cyclamate and aspartame.

7. The process of claim 4, **characterized** in that the hydrophilic nonionic surfactants are a polyoxyethylene derivative of a sorbitan mono fatty ester and a block copolymer of propylene oxide and ethylene oxide having an HLB of between 9 and 30.

8. The process of claim 4, **characterized** in that the sorbitan mono fatty ester surfactant is a polyoxyethylene derivative of a sorbitan fatty acid ester wherein the ester-forming fatty acid is selected from the group consisting of lauric acid, palmitic acid, oleic acid, and stearic acid.

9. The process of claim 8, **characterized** in that the sorbitan fatty acid ester surfactant and the block copolymer are present in a weight ratio of 1:2 to 1:200.

10. The process of claim 9, **characterized** in that the emulsion has a pH of 3.5 to 3.9 and that the concentration of the aluminum ions is 500 ppm.

11. The process of claim 10, **characterized** in that the sorbitan fatty acid ester is a polyoxyethylene derivative of sorbitan monolaurate having an HLB of 9 to 18 and that the block copolymer of propylene oxide and ethylene oxide has an HLB of 15 to 30.

12. The process of claim 4, **characterized** in that the aluminum ions are present at 500 to 2 000 ppm and that the sorbitan fatty acid ester is a polyoxyethylene derivative of sorbitan monolaurate having an HLB of 17 and that the blocked copolymer of propylene oxide and ethylene oxide has an HLB of 22.

13. The process of claim 12, **characterized** in that a dental rinse or mouthwash is prepared.

14. The process of claim 13, **characterized** in that the sorbitan fatty acid ester constitutes 0.1 to 5 percent by weight of the product and that the block copolymer of propylene oxide and ethylene oxide constitutes 1 to 20 percent by weight of the product.

15. The process of claim 14, **characterized** in that the polyoxyethylene derivative of a sorbitan monolaurate and the block copolymer of propylene oxide and ethylene oxide have an HLB of 17 and 22, respectively and that they are used in a weight ratio of 1:4 to 1: 50.

16. The process of claim 1, **characterized** in that additionally water-soluble fluoride-containing salts are added to the mixture.

17. The process of claim 12, **characterized** in that 100 to 1 000 ppm fluoride ions are added.

18. A process for preparing a palatable anticariogenic emulsion adapted for application to teeth, said emulsion having a pH of 2.5 to 5.0, **characterized** by mixing
   10 to 50 000 ppm aluminum ions capable of reacting with the teeth,
   a sweetener,
   0.1 to 5.0 weight percent of a flavour oil,
   water, and
   a surfactant selected from the group consisting of polyalkoxylated anionic surfactants, cationic polyalkoxyamines, quaternary ammonium salts, and amphoteric surfactants derived from amino acids selected from the group consisting of glycine, cysteine, and phenylalanine, said surfactants being present in an amount effective to stabilize the anticariogenic emulsion.

19. The process of claim 18 **characterized** in that a product selected from the group consisting of a dental rinse, a mouthwash, a toothpaste, a prophylaxis paste, a chewing gum, and a lozenge is prepared

20. The process of claim 18 **characterized** in that an ingredient selected from the group consisting of 1 to

24

90 weight percent of a humectant selected from glycerin and sugar alcohols, and that the aluminum ions are present at a level of 250 to 10 000 rpm.

**21.** The process of claim 20, **characterized** in that the humectant is selected from the group consisting of glycerin, xylitol, mannitol and sorbitol.

**22.** The process of claim 20, **characterized** in that the sweetener comprises a non-cariogenic sweetener selected from the group consisting of saccharin, cyclamate and aspartame.

**23.** The process of claim 18, **characterized** in that the surfactant has an HLB of between 9 and 30.

**24.** The process of claim 23, **characterized** in that the surfactant is selected from the group consisting of polyalkoxylated anionic and polyalkoxylated cationic surfactants.

**25.** The process of claim 23, **characterized** in that the surfactant is a quaternary ammonium salt.

**26.** The process of claim 23, **characterized** in that the surfactant is a derivative of an amino acid selected from the group consisting of glycine, cysteine and phenylalanine.

## Revendications

**1.** Emulsion anticarie agréable au palais, adaptée pour l'application aux dents, ladite émulsion ayant un pH de 2,5 à 5,0 et comprenant
10 à 50 000 ppm d'ions aluminium aptes à réagir avec les dents,
un édulcorant,
0,1 à 5,0 pour cent en poids d'une essence de parfum
de l'eau, et
des agents de surface non-ioniques hydrophiles, comprenant (1) 0,1 à 5 pour cent en poids de dérivés polyoxyéthylénés d'esters d'acide gras de sorbitan et (2) 0,1 à 20 pour cent en poids de copolymères séquencés d'oxyde de propylène et d'oxyde d'éthylène, lesdits agents de surface étant présents en une quantité efficace pour stabiliser l'émulsion anticarie.

**2.** Emulsion anticarie selon la revendication 1, sous la forme d'un produit choisi parmi un produit de rinçage dentaire, une eau dentifrice, une pâte dentifrice, une pâte prophylactique, une gomme à mâcher et une pastille.

**3.** Emulsion selon la revendication 1, dans laquelle les ions aluminium sont dérivés d'un sel d'aluminium hydrosoluble choisi parmi le sulfate de potassium et d' aluminium, le chlorure d'aluminium, le sulfate de sodium et d'aluminium, le phosphate de sodium et d'aluminium,le sulfate d'aluminium et le nitrate d'aluminium.

**4.** Emulsion selon la revendication 1, comprenant en outre un constituant choisi parmi 1 à 90 pour cent en poids d'un agent humectant sélectionné parmi la glycérine et les alcools de sucre, et où les ions aluminium sont présents à une teneur de 250 à 10 000 ppm.

**5.** Emulsion selon la revendication 4, dans laquelle l'agent humectant est choisi parmi la glycérine, le xylitol, le mannitol et le sorbitol.

**6.** Emulsion selon la revendication 4, dans laquelle l'édulcorant comprend un édulcorant non-cariogène choisi parmi la saccharine, le cyclamate et l'aspartame.

**7.** Emulsion selon la revendication 4, dans laquelle les agents de surface non-ioniques hydrophiles sont un dérivé polyoxyéthyléné d'un monoester gras de sorbitan et un copolymère séquencé d'oxyde de propylène et d' oxyde d'éthylène ayant un HLB compris entre 9 et 30.

**8.** Emulsion selon la revendication 4, dans laquelle l'agent de surface monoester gras de sorbitan est un dérivé polyoxyéthyléné d'un ester d'acide gras de sorbitan dans lequel l'acide gras formant l'ester est choisi parmi l'acide laurique, l'acide palmitique, l' acide oléique et l'acide stéarique.

9. Emulsion selon la revendication 8, dans laquelle l'agent de surface ester d'acide gras de sorbitan et le copolymère séquencé sont présents dans un rapport en poids de 1:2 à 1:200.

10. Emulsion selon la revendication 9, ayant un pH de 3,5 à 3,9 avec des ions aluminium à raison de 500 ppm.

11. Emulsion selon la revendication 10, dans laquelle l'ester d'acide gras de sorbitan est un dérivé polyoxyéthyléné de monolaurate de sorbitan ayant un HLB de 9 à 18 et le copolymère séquencé d'oxyde de propylène et d'oxyde d'éthylène a un HLB de 15 à 30.

12. Emulsion selon la revendication 4, dans laquelle les ions aluminium sont présents à raison de 500 à 2000 ppm et l'ester d'acide gras de sorbitan est un dérivé polyoxyéthyléné de monolaurate de sorbitan ayant un HLB de 17 et le copolymère séquencé d'oxyde de propylène et d'oxyde d'éthylène a un HLB de 22.

13. Emulsion selon la revendication 12, sous la forme d'un produit de rinçage dentaire ou d'une eau dentifrice.

14. Produit selon la revendication 13, dans lequel l'ester d'acide gras de sorbitan constitue 0,1 à 5 pour cent en poids du produit et le copolymère séquencé d'oxyde de propylène et d'oxyde d'éthylène constitue 1 à 20 pour cent en poids du produit.

15. Produit selon la revendication 14, dans lequel le dérivé polyoxyéthyléné d'un monolaurate de sorbitan et le copolymère séquencé d'oxyde de propylène et d'oxyde d'éthylène ont un HLB de 17 et 22 respectivement et sont utilisés dans un rapport en poids de 1:4 à 1:50.

16. Emulsion selon la revendication 1, contenant en plus des sels hydrosolubles contenant du fluor.

17. Emulsion selon la revendication 12, contenant en plus 100 à 1000 ppm d'ions fluorure.

18. Emulsion anticarie agréable au palais, adaptée pour l'application aux dents, ladite émulsion ayant un pH de 2,5 à 5,0 et comprenant
10 à 50 000 ppm d'ions aluminium aptes à réagir avec les dents,
un édulcorant,
0,1 à 5,0 pour cent en poids d'une essence de flaveur,
de l'eau, et
un agent de surface choisi parmi les agents de surface anioniques polyalcoxylés, les polyalcoxyamines cationiques, les sels d'ammonium quaternaire, et des agents de surface amphotères dérivés d'acides aminés sélectionnés parmi la glycine, la cystéine et la phénylalanine, lesdits agents de surface étant présents en une quantité efficace pour stabiliser l'émulsion anticarie.

19. Emulsion anticarie selon la revendication 18, sous la forme d'un produit choisi parmi un produit de rinçage dentaire, une eau dentifrice, une pâte dentifrice, une pâte prophylactique, une gomme à mâcher et une pastille.

20. Emulsion selon la revendication 18, comprenant en outre un constituant choisi parmi 1 à 90 pour cent en poids d'un agent humectant sélectionné parmi la glycérine et les alcools de sucre, et où les ions aluminium sont présents à une teneur de 250 à 10 000 ppm.

21. Emulsion selon la revendication 20, dans laquelle l'agent humectant est choisi parmi la glycérine, le xylitol, le mannitol et le sorbitol.

22. Emulsion selon la revendication 20, dans laquelle l'édulcorant comprend un édulcorant non-cariogène choisi parmi la saccharine, le cyclamate et l'aspartame.

23. Emulsion selon la revendication 20, dans laquelle l'agent de surface a un HLB compris entre 9 et 30.

24. Emulsion selon la revendication 23, dans laquelle l'agent de surface est choisi parmi les agents de surface anioniques polyalcoxylés et les agents de surface cationiques polyalcoxylés.

25. Emulsion selon la revendication 23, dans laquelle l'agent de surface est un sel d'ammonium quaternaire.

26. Emulsion selon la revendication 23, dans laquelle l'agent de surface est un dérivé d'un acide aminé choisi parmi la glycine, la cystéine et la phénylalanine.

**Revendications pour l'Etat contractant suivant : ES**

1. Procédé pour la préparation d'une émulsion anticarie agréable au palais, adaptée pour l'application aux dents, ladite émulsion ayant un pH de 2,5 à 5,0,
   caractérisé en ce qu'on mélange
   10 à 50 000 ppm d'ions aluminium aptes à réagir avec les dents,
   un édulcorant,
   0,1 à 5,0 pour cent en poids d'une essence de flaveur,
   de l'eau, et
   des agents de surface non-ioniques hydrophiles, comprenant (1) 0,1 à 5 pour cent en poids de dérivés polyoxyéthylénés d'esters d'acide gras de sorbitan et (2) 0,1 à 20 pour cent en poids de copolymères séquencés d'oxyde de propylène et d'oxyde d'éthylène, lesdits agents de surface étant présents en une quantité efficace pour stabiliser l'émulsion anticarie.

2. Procédé selon la revendication 1, caractérisé en ce qu'on prépare un produit choisi parmi un produit de rinçage dentaire, une eau dentifrice, une pâte dentifrice, une pâte prophylactique, une gomme à mâcher et une pastille.

3. Procédé selon la revendication 1, caractérisé en ce que les ions aluminium sont dérivés d'un sel d'aluminium hydrosoluble choisi parmi le sulfate de potassium et d'aluminium, le chlorure d'aluminium, le sulfate de sodium et d'aluminium, le phosphate de sodium et d'aluminium, le sulfate d'aluminium et le nitrate d'aluminium.

4. Procédé selon la revendication 1, caractérisé en ce qu'on ajoute au mélange un constituant choisi parmi 1 à 90 pour cent en poids d'un agent humectant sélectionné parmi la glycérine et les alcools de sucre, et dans lequel les ions aluminium sont présents à une teneur de 250 à 10 000 ppm.

5. Procédé selon la revendication 4, caractérisé en ce que l'agent humectant est choisi parmi la glycérine, le xylitol, le mannitol et le sorbitol.

6. Procédé selon la revendication 4, caractérisé en ce que l'édulcorant comprend un édulcorant non-cariogène choisi parmi la saccharine, le cyclamate et l'aspartame.

7. Procédé selon la revendication 4, caractérisé en ce que les agents de surface non-ioniques hydrophiles sont un dérivé polyoxyéthyléné d'un monoester gras de sorbitan et un copolymère séquencé d'oxyde de propylène et d'oxyde d'éthylène ayant un HLB compris entre 9 et 30.

8. Procédé selon la revendication 4, caractérisé en ce que l'agent de surface monoester gras de sorbitan est un dérivé polyoxyéthyléné d'un ester d'acide gras de sorbitan dans lequel l'acide gras formant l'ester est choisi parmi l'acide laurique, l'acide palmitique, l'acide oléique et l'acide stéarique.

9. Procédé selon la revendication 8, caractérisé en ce que l'agent de surface ester d'acide gras de sorbitan et le copolymère séquencé sont présents dans un rapport en poids de 1:2 à 1:200.

10. Procédé selon la revendication 9, caractérisé en ce que l'émulsion a un pH de 3,5 à 3,9 et la concentration des ions aluminium est de 500 ppm.

11. Procédé selon la revendication 10, caractérisé en ce que l'ester d'acide gras de sorbitan est un dérivé polyoxyéthyléné de monolaurate de sorbitan ayant un HLB de 9 à 18 et le copolymère séquencé d'oxyde de propylène et d'oxyde d'éthylène a un HLB de 15 à 30.

27

**12.** Procédé selon la revendication 4, caractérisé en ce que les ions aluminium sont présents à raison de 500 à 2000 ppm et l'ester d'acide gras de sorbitan est un dérivé polyoxyéthyléné de monolaurate de sorbitan ayant un HLB de 17 et le copolymère séquencé d'oxyde de propylène et d'oxyde d'éthylène a un HLB de 22.

**13.** Procédé selon la revendication 12, caractérisé en ce qu'on prépare un produit de rinçage dentaire ou une eau dentifrice.

**14.** Procédé selon la revendication 13, caractérisé en ce que l'ester d'acide gras de sorbitan constitue 0,1 à 5 pour cent en poids du produit et le copolymère séquencé d'oxyde de propylène et d'oxyde d'éthylène constitue 1 à 20 pour cent en poids du produit.

**15.** Procédé selon la revendication 14, caractérisé en ce que le dérivé polyoxyétnyléné d'un monolaurate de sorbitan et le copolymère séquencé d'oxyde de propylène et d'oxyde d'éthylène ont un HLB de 17 et 22 respectivement et sont utilisés dans un rapport en poids de 1:4 à 1:50.

**16.** Procédé selon la revendication 1, caractérisé en ce qu'on ajoute en plus au mélange des sels hydrosolubles contenant du fluor.

**17.** Procédé selon la revendication 12, caractérisé en ce qu'on ajoute 100 à 1000 ppm d'ions fluorure.

**18.** Procédé pour la préparation d'une émulsion anticarie agréable au palais, adaptée pour l'application aux dents, ladite émulsion ayant un pH de 2,5 à 5,0,
caractérisé en ce qu'on mélange
   10 à 50 000 ppm d'ions aluminium aptes à réagir avec les dents,
   un édulcorant,
   0,1 à 5,0 pour cent en poids d'une essence de flaveur,
   de l'eau, et
   un agent de surface choisi parmi les agents de surface anioniques polyalcoxylés, les polyalcoxyamines cationiques, les sels d'ammonium quaternaire, et des agents de surface amphotères dérivés d'acides aminés sélectionnés parmi la glycine, la cystéine et la phénylalanine, lesdits agents de surface étant présents en une quantité efficace pour stabiliser l'émulsion anticarie.

**19.** Procédé selon la revendication 18, caractérisé en ce qu'on prépare un produit choisi parmi un produit de rinçage dentaire, une eau dentifrice, une pâte dentifrice, une pâte prophylactique, une gomme à mâcher et une pastille.

**20.** Procédé selon la revendication 18, caractérisé en ce qu'un constituant choisi parmi 1 à 90 pour cent en poids d'un agent humectant sélectionné parmi la glycérine et les alcools de sucre est ajouté et les ions aluminium sont présents à une teneur de 250 à 10 000 ppm.

**21.** Procédé selon la revendication 20, caractérisé en ce que l'humectant est choisi parmi la glycérine, le xylitol, le mannitol et le sorbitol.

**22.** Procédé selon la revendication 20, caractérisé en ce que l'édulcorant comprend un édulcorant non-cariogène choisi parmi la saccharine, le cyclamate et l'aspartame.

**23.** Procédé selon la revendication 18, caractérisé en ce que l'agent de surface a un HLB compris entre 9 et 30.

**24.** Procédé selon la revendication 23, caractérisé en ce que l'agent de surface est choisi parmi les agents de surface anioniques polyalcoxylés et les agents de surface cationiques polyalcoxylés.

**25.** Procédé selon la revendication 23, caractérisé en ce que l'agent de surface est un sel d'ammonium quaternaire.

**26.** Procédé selon la revendication 23, caractérisé en ce que l'agent de surface est un dérivé d'un acide aminé choisi parmi la glycine, la cystéine et la phénylalanine.

**Patentansprüche**

1. Schmackhafte antikariogene Emulsion, geeignet zur Aufbringung auf die Zähne, dadurch **gekennzeichnet,** daß die Emulsion einen pH von 2,5 bis 5,0 besitzt und 10 bis 50.000 ppm Aluminiumionen, die mit den Zähnen reagieren können, einen Süßstoff, 0,1 bis 5,0 Gewichtsprozent eines Aromaöls, Wasser, und hydrophile, nicht-ionische oberflächenaktive Mittel, umfassend
(1) 0,1 bis 5 Gewichtsprozent Polyoxyethylen-Derivate von Sorbitan-Fettsäure-Estern und (2) 0,1 bis 20 Gewichtsprozent Block-Copolymere von Propylenoxid und Ethylenoxid, enthält, wobei die oberflächenaktiven Mittel in einer Menge vorliegen, die wirksam ist, die antikariogene Emulsion zu stabilisieren.

2. Antikariogene Emulsion nach Anspruch 1, dadurch **gekennzeichnet,** daß die Form eines Produkts aus der Gruppe bestehend aus Zahnspülung, Mundwasser, Zahnpasta, prophylaktischer Paste, Kaugummi und Pastille, ausgewählt ist.

3. Emulsion nach Anspruch 1, dadurch **gekennzeichnet,** daß die Aluminiumionen sich von einem wasserlöslichen Aluminiumsalz, ausgewählt aus der Gruppe bestehend aus Aluminium-Kalium-Sulfat, Aluminiumchlorid, Aluminium-Natrium-Sulfat, Aluminium-Natrium-Phosphat, Aluminiumsulfat und Aluminiumnitrat, ableiten.

4. Emulsion nach Anspruch 1, dadurch **gekennzeichnet,** daß sie weiterhin einen Inhaltsstoff, ausgewählt aus den Gruppen bestehend aus 1 bis 90 Gewichtsprozent eines Feuchthaltemittels, ausgewählt aus Glyzerin und Zuckeralkoholen enthält, und daß die Aluminiumionen in einer Konzentration von 250 bis 10.000 ppm vorliegen.

5. Emulsion nach Anspruch 4, dadurch **gekennzeichnet,** daß das Feuchthaltemittel aus der Gruppe bestehend aus Glyzerin, Xylit, Mannit und Sorbit ausgewählt ist.

6. Emulsion nach Anspruch 4, dadurch **gekennzeichnet,** daß der Süßstoff einen nicht-kariogenen Süßstoff, ausgewählt aus der Gruppe bestehend aus Saccharin, Cyclamat und Aspartam, enthält.

7. Emulsion nach Anspruch 4, dadurch **gekennzeichnet,** daß die hydrophilen, nicht-ionischen, oberflächenaktiven Mittel ein Polyoxyethylen-Derivat eines Sorbitan-Monofettsäure-Esters und ein Block-Copolymeres aus Propylenoxid und Ethylenoxid mit einem HLB zwischen 9 und 30 sind.

8. Emulsion nach Anspruch 4, dadurch **gekennzeichnet,** daß das oberflächenaktive Mittel Sorbitan-Monofettsäure-Ester ein Polyoxyethylenderivat eines Sorbitan-Fettsäure-Esters ist, worin die esterbildende Fettsäure aus der Gruppe bestehend aus Laurinsäure, Palmitinsäure, Ölsäure und Stearinsäure ausgewählt ist.

9. Emulsion nach Anspruch 8, dadurch **gekennzeichnet,** daß das oberflächenaktive Mittel Sorbitan-Fettsäure-Ester und das Block-Copolymere in einem Gewichtsverhältnis von 1:2 bis 1:200 vorliegen.

10. Emulsion nach Anspruch 9, dadurch **gekennzeichnet,** daß sie einen pH von 3,5 bis 3,9 besitzt und die Aluminiumionen zu 500 ppm vorliegen.

11. Emulsion nach Anspruch 10, dadurch **gekennzeichnet,** daß der Sorbitan-Fettsäure-Ester ein Polyoxyethylenderivat von Sorbitan-Monolaurat mit einem HLB von 9 bis 18 ist und das Block-Copolymere von Propylenoxid und Ethylenoxid einen HLB von 15 bis 30 besitzt.

12. Emulsion nach Anspruch 4, dadurch **gekennzeichnet,** daß die Aluminiumionen in einer Konzentration von 500 bis 2.000 ppm vorliegen und der Sorbitan-Fettsäure-Ester ein Polyoxyethylenderivat von Sorbitan-Monolaurat mit einem HLB von 17 ist, und das Block-Copolymere von Propylenoxid und Ethylenoxid einen HLB von 22 besitzt.

13. Emulsion nach Anspruch 12, dadurch **gekennzeichnet,** daß sie in Form einer Zahnspülung oder eines Mundwassers vorliegt.

14. Produkt nach Anspruch 13, dadurch **gekennzeichnet,** daß der Sorbitan-Fettsäure-Ester 0,1 bis 5

29

Gewichtsprozent des Produkts ausmacht und das Block-Copolymere von Propylenoxid und Ethylenoxid 1 bis 20 Gewichtsprozent des Produkts ausmacht.

**15.** Produkt nach Anspruch 14, dadurch **gekennzeichnet,** daß das Polyoxyethylenderivat eines Sorbitan-Monolaurats und das Block-Copolymere von Propylenoxid und Ethylenoxid einen HLB von 17 bzw. 22 besitzen und in einem Gewichtsverhältnis von 1:4 bis 1:50 verwendet werden.

**16.** Emulsion nach Anspruch 1, dadurch **gekennzeichnet,** daß sie zusätzlich wasserlösliche fluoridhaltige Salze enthält.

**17.** Emulsion nach Anspruch 12, dadurch **gekennzeichnet,** daß sie zusätzlich 100 bis 1.000 ppm Fluoridionen enthält.

**18.** Schmackhafte antikariogene Emulsion, geeignet zur Anwendung auf Zähne, dadurch **gekennzeichnet,** daß die Emulsion einen pH von 2,5 bis 5,0 besitzt und 10 bis 50.000 ppm Aluminiumionen, die mit den Zähnen reagieren können, einen Süßstoff, 0,1 bis 5,0 Gewichtsprozent eines Aromaöls, Wasser, und ein oberflächenaktives Mittel, ausgewählt aus der Gruppe bestehend aus polyalkoxylierten, anionischen, oberflächenaktiven Mitteln, kationischen Polyalkoxyaminen, quaternären Ammoniumsalzen und amphotären, oberflächenaktiven Mitteln, abgeleitet von Aminosäuren, ausgewählt aus der Gruppe bestehend aus Glyzin, Cystein und Phenylalanin, enthält, wobei die oberflächenaktiven Mittel in einer Menge vorliegen, die wirksam ist, die antikariogene Emulsion zu stabilisieren.

**19.** Antikariogene Emulsion nach Anspruch 18, dadurch **gekennzeichnet,** daß sie in Form eines Produkts, ausgewählt aus der Gruppe bestehend aus Zahnspülung, Mundwasser, Zahnpasta, prophylaktischer Paste, Kaugummi und Pastille, vorliegt.

**20.** Emulsion nach Anspruch 18, dadurch **gekennzeichnet,** daß sie weiterhin einen Inhaltsstoff ausgewählt aus der Gruppe bestehend aus 1 bis 90 Gewichtsprozent eines Feuchthaltemittels, ausgewählt aus Glyzerin und Zuckeralkoholen, enthält und daß die Aluminiumionen in einer Konzentration von 250 bis 10.000 ppm vorliegen.

**21.** Emulsion nach Anspruch 20, dadurch **gekennzeichnet,** daß das Feuchthaltemittel aus der Gruppe bestehend aus Glyzerin, Xylit, Mannit und Sorbit ausgewählt ist.

**22.** Emulsion nach Anspruch 20, dadurch **gekennzeichnet,** daß der Süßstoff einen nicht-kariogenen Süßstoff, ausgewählt aus der Gruppe bestehend aus Saccharin, Cyclamat und Aspartam, umfaßt.

**23.** Emulsion nach Anspruch 20, dadurch **gekennzeichnet,** daß das oberflächenaktive Mittel einen HLB zwischen 9 und 30 besitzt.

**24.** Emulsion nach Anspruch 23, dadurch **gekennzeichnet,** daß das oberflächenaktive Mittel aus der Gruppe bestehend aus polyalkoxylierten, anionischen und polyalkoxylierten, kationischen, oberflächenaktiven Mitteln ausgewählt ist.

**25.** Emulsion nach Anspruch 23, dadurch **gekennzeichnet,** daß das oberflächenaktive Mittel ein quaternäres Ammoniumsalz ist.

**26.** Emulsion nach Anspruch 23, dadurch **gekennzeichnet,** daß das oberflächenaktive Mittel ein Derivat einer Aminosäure, ausgewählt aus der Gruppe bestehend aus Glyzin, Cystein und Phenylalanin, ist.

**Patentansprüche für folgenden Vertragsstaat : ES**

**1.** Verfahren zur Herstellung einer schmackhaften antikariogenen Emulsion, geeignet zur Aufbringung auf die Zähne, wobei die Emulsion einen pH von 2,5 bis 5,0 besitzt, dadurch **gekennzeichnet,** daß man 10 bis 50.000 ppm Aluminiumionen, die mit den Zähnen reagieren können, einen Süßstoff, 0,1 bis 5,0 Gewichtsprozent eines Aromaöls, Wasser, und hydrophile, nicht-ionische, oberflächenaktive Mittel, umfassend
(1) 0,1 bis 5 Gewichtsprozent Polyoxyethylen-Derivate von Sorbitan-Fettsäure-Estern und (2) 0,1 bis 20

30

EP 0 270 977 B1

Gewichtsprozent Block-Copolymere von Propylenoxid und Ethylenoxid mischt, wobei die oberflächenaktiven Mittel in einer Menge vorliegen, die wirksam ist, die antikariogene Emulsion zu stabilisieren.

2. Verfahren nach Anspruch 1, dadurch **gekennzeichnet,** daß ein Produkt, ausgewählt aus der Gruppe bestehend aus Zahnspülung, Mundwasser, Zahnpasta, prophylaktischer Paste, Kaugummi und Pastille, hergestellt wird.

3. Verfahren nach Anspruch 1, dadurch **gekennzeichnet,** daß die Aluminiumionen sich von einem wasserlöslichen Aluminiumsalz, ausgewählt aus der Gruppe bestehend aus Aluminium-Kalium-Sulfat, Aluminiumchlorid, Aluminium-Natrium-Sulfat, Aluminium-Natrium-Phosphat, Aluminiumsulfat und Aluminiumnitrat, ableiten.

4. Verfahren nach Anspruch 1, dadurch **gekennzeichnet,** daß man zu dem Gemisch einen Inhaltsstoff, ausgewählt aus der Gruppe bestehend aus 1 bis 90 Gewichtsprozent eines Feuchthaltemittels, ausgewählt aus Glyzerin und Zuckeralkoholen, zugibt, und daß die Aluminiumionen in einer Konzentration von 250 bis 10.000 ppm vorliegen.

5. Verfahren nach Anspruch 4, dadurch **gekennzeichnet,** daß das Feuchthaltemittel aus der Gruppe bestehend aus Glyzerin, Xylit, Mannit und Sorbit ausgewählt wird.

6. Verfahren nach Anspruch 4, dadurch **gekennzeichnet,** daß der Süßstoff einen nicht-kariogenen Süßstoff, ausgewählt aus der Gruppe bestehend aus Saccharin, Cyclamat und Aspartam, umfaßt.

7. Verfahren nach Anspruch 4, dadurch **gekennzeichnet,** daß die hydrophilen, nicht-ionischen, oberflächenaktiven Mittel ein Polyoxyethylen-Derivat eines Sorbitan-Monofettsäure-Esters und ein Block-Copolymeres von Propylenoxid und Ethylenoxid mit einem HLB zwischen 9 und 30 sind.

8. Verfahren nach Anspruch 4, dadurch **gekennzeichnet,** daß das oberflächenaktive Mittel Sorbitan-Monofettsäure-Ester ein Polyoxyethylenderivat eines Sorbitan-Fettsäure-Esters ist, worin die esterbildende Fettsäure aus der Gruppe bestehend aus Laurinsäure, Palmitinsäure, Ölsäure und Stearinsäure ist.

9. Verfahren nach Anspruch 8, dadurch **gekennzeichnet,** daß das oberflächenaktive Mittel Sorbitan-Fettsäure-Ester und das Block-Copolymere in einem Gewichtsverhältnis von 1:2 bis 1:200 vorliegen.

10. Verfahren nach Anspruch 9, dadurch **gekennzeichnet,** daß die Emulsion einen pH von 3,5 bis 3,9 besitzt und daß die Konzentration der Aluminiumionen 500 ppm ist.

11. Verfahren nach Anspruch 10, dadurch **gekennzeichnet,** daß der Sorbitan-Fettsäure-Ester ein Polyoxyethylenderivat eines Sorbitan-Monolaurats mit einem HLB von 9 bis 18 ist und daß das Block-Copolymere von Propylenoxid und Ethylenoxid einen HLB von 15 bis 30 besitzt.

12. Verfahren nach Anspruch 4, dadurch **gekennzeichnet,** daß die Aluminiumionen in einer Konzentration von 500 bis 2.000 ppm vorliegen und daß der Sorbitan-Fettsäure-Ester ein Polyoxyethylenderivat von Sorbitan-Monolaurat mit einem HLB von 17 ist, und daß das Block-Copolymere von Propylenoxid und Ethylenoxid einen HLB von 22 besitzt.

13. Verfahren nach Anspruch 12, dadurch **gekennzeichnet,** daß eine Zahnspülung oder ein Mundwasser hergestellt wird.

14. Verfahren nach Anspruch 13, dadurch **gekennzeichnet,** daß der Sorbitan-Fettsäure-Ester 0,1 bis 5 Gewichtsprozent des Produkts ausmacht und daß das Block-Copolymere von Propylenoxid und Ethylenoxid 1 bis 20 Gewichtsprozent des Produkts ausmacht.

15. Verfahren nach Anspruch 14, dadurch **gekennzeichnet,** daß das Polyoxyethylenderivat eines Sorbitan-Monolaurats und das Block-Copolymere von Propylenoxid und Ethylenoxid einen HLB von 17 bzw. 22 besitzen und daß sie in einem Gewichtsverhältnis von 1:4 bis 1:50 verwendet werden.

31

**16.** Verfahren nach Anspruch 1, dadurch **gekennzeichnet,** daß weiterhin wasserlösliche fluoridhaltige Salze dem Gemisch zugesetzt werden.

**17.** Verfahren nach Anspruch 12, dadurch **gekennzeichnet,** daß 100 bis 1.000 ppm Fluoridionen zugesetzt werden.

**18.** Verfahren zur Herstellung einer schmackhaften antikariogenen Emulsion, geeignet zur Aufbringung auf die Zähne, wobei die Emulsion einen pH von 2,5 bis 5,0 besitzt, dadurch **gekennzeichnet,** daß man 10 bis 50.000 ppm Aluminiumionen, die mit den Zähnen reagieren können, einen Süßstoff, 0,1 bis 5,0 Gewichtsprozent eines Aromaöls, Wasser und ein oberflächenaktives Mittel, ausgewählt aus der Gruppe bestehend aus polyalkoxylierten, anionischen, oberflächenaktiven, Mitteln, kationischen Polyalkoxyaminen, quaternären Ammoniumsalzen und amphotären, oberflächenaktiven Mitteln, abgeleitet von Aminosäuren, ausgewählt aus der Gruppe bestehend aus Glyzin, Cystein und Phenylalanin mischt, wobei die oberflächenaktiven Mittel in einer Menge vorliegen, die wirksam ist, die antikariogene Emulsion zu stabilisieren.

**19.** Verfahren nach Anspruch 18, dadurch **gekennzeichnet,** daß ein Produkt, ausgewählt aus der Gruppe bestehend aus Zahnspülung, Mundwasser, Zahnpasta, prophylaktischer Paste, Kaugummi und Pastille, hergestellt wird.

**20.** Verfahren nach Anspruch 18, dadurch **gekennzeichnet,** daß ein Inhaltsstoff, ausgewählt aus der Gruppe bestehend aus 1 bis 90 Gewichtsprozent eines Feuchthaltemittels, ausgewählt aus Glyzerin und Zuckeralkoholen, und Aluminiumionen in einer Konzentration von 250 bis 10.000 ppm vorliegen.

**21.** Verfahren nach Anspruch 20, dadurch **gekennzeichnet,** daß das Feuchthaltemittel aus der Gruppe bestehend aus Glyzerin, Xylit, Mannit und Sorbit ausgewählt wird.

**22.** Verfahren nach Anspruch 20, dadurch **gekennzeichnet,** daß der Süßstoff einen nicht-kariogenen Süßstoff, ausgewählt aus der Gruppe bestehend aus Saccharin, Cyclamat und Aspartam, umfaßt.

**23.** Verfahren nach Anspruch 18, dadurch **gekennzeichnet,** daß das oberflächenaktive Mittel einen HLB zwischen 9 und 30 besitzt.

**24.** Verfahren nach Anspruch 23, dadurch **gekennzeichnet,** daß das oberflächenaktive Mittel aus der Gruppe bestehend aus polyalkoxylierten, anionischen und polyalkoxylierten, kationischen, oberflächenaktiven Mitteln ausgewählt wird.

**25.** Verfahren nach Anspruch 23, dadurch **gekennzeichnet,** daß das oberflächenaktive Mittel ein quaternäres Ammoniumsalz ist.

**26.** Verfahren nach Anspruch 23, dadurch **gekennzeichnet,** daß das oberflächenaktive Mittel ein Derivat einer Aminosäure, ausgewählt aus der Gruppe bestehend aus Glyzin, Cystein und Phenylalanin, ist.